# EUROPEAN PATENT APPLICATION

(11) **EP 4 385 522 A1**
(43) Date of publication of application: **19.06.2024**
(21) Application number: 22855468.9
(22) Date of filing: 10.08.2022
(51) Int. Cl.: A61K 47/68, C07K 16/28, A61P 35/00

(54) **MICROTUBULE INHIBITOR-BASED ANTIBODY-DRUG CONJUGATE**

(30) Priority: 13.08.2021 CN 202110929642
(71) Applicant: Kunshan Xinyunda Biotech Co., Ltd., Kunshan, Jiangsu 215300 (CN)
(72) Inventor: DING, Hui, Suzhou, Jiangsu 215300 (CN); YU, Haiyong, Suzhou, Jiangsu 215300 (CN); XU, Yunlei, Suzhou, Jiangsu 215300 (CN); LAO, Fang, Suzhou, Jiangsu 215300 (CN); LIU, Yan, Suzhou, Jiangsu 215300 (CN); ZHANG, Xidong, Suzhou, Jiangsu 215300 (CN); RONG, Pengfei, Suzhou, Jiangsu 215300 (CN); KE, Tianyi, Suzhou, Jiangsu 215300 (CN)
(74) Representative: van Dam, Vincent
(86) International application number: PCT/CN2022/111443
(87) International publication number: WO 2023/016488

(57) **Abstract**

The present invention relates to the technical field of medicines, and in particular to a microtubule inhibitor-based antibody-drug conjugate. Specifically, the present invention relates to a compound represented by formula (I), a pharmaceutically acceptable salt thereof, a stereoisomer thereof, or a solvate of the compound, the pharmaceutically acceptable salt thereof, or the stereoisomer thereof, and also relates to an antibody-drug conjugate formed by linking, via a thioether linkage, a targeting moiety and the compound represented by formula (I), the pharmaceutically acceptable salt thereof, the stereoisomer thereof, or the solvate of the conjugate, the pharmaceutically acceptable salt thereof or the stereoisomer thereof.

L¹-L²-L³-L⁴-D (I)

## Description

### TECHNICAL FIELD

The present disclosure relates to an antibody-drug conjugate (ADC) that binds a human oncology antigen target and/or provides anti-tubulin drug activity. The present disclosure further relates to a method and composition for treating a cancer that express a tumor antigen target and/or is treatable by disrupting tubulin.

### BACKGROUND

In the field of tumor treatment, antibody-drug conjugates (ADCs) obtained by conjugating monoclonal antibodies to toxin small molecules have become a hot spot in targeted tumor therapy. An antibody-drug conjugate (ADC) is obtained by linking a biologically active small molecule drug to a monoclonal antibody via a chemical linkage. The monoclonal antibody serves as a vector to transfer the small molecule drug into a target cell. Research on ADCs can be traced back to the 1980s, but it was not until 2000 that the first ADC (trade name: Mylotarg, developed by Pfizer) was approved by the FDA for the treatment of acute myeloid leukemia, and was withdrawn from the market in 2010 due to fatal toxicity caused by immature technology. Takeda/SeattleGenetics improved its original technology and developed brentuximabvedotin (SGN-35, trade name: Adcetris), which was approved by the FDA in 2011 for the treatment of Hodgkin lymphoma and systemic anaplastic large cell lymphoma. In 2013, Ado-trastuzumabemtansine (T-DM1, trade name: Kadcyla), jointly developed by Genentech/ImmunoGen, was approved by the FDA for the treatment of HER2-positive breast cancer, becoming the first ADC targeting solid tumors. Currently, there are 12 types of ADCs approved for marketing, among which the products that have attracted attention for their effects include IMMU-132, ds-8210a developed by Daiichi Sankyo Company Limited, etc.

An ADC consists of an antibody, a small molecule toxin and a linker. The small molecule toxin is covalently conjugated to the antibody via the linker. The antibody (such as a monoclonal antibody) can specifically recognize a specific target on the surface of tumor cells and can then guide the ADC to the surface of cancer cells, allowing the ADC to enter the cells by endocytosis and kill the cells.

Small molecule toxins used for ADCs comprise two categories: DNA damaging agents and microtubule inhibitors. The former includes camptothecins, and the latter includes aplysiatoxin and its auristatins (MMAE, MMAF, MMAD), as well as maytansine and maytansinoids (DM1, DM2, DM3, DM4). Currently, the vast majority of ADCs under clinical development use tubulin inhibitors. For example, the small molecule toxin used in Kadcyla is DM1. In 2021, Eisai and Bristol-Myers Squibb (BMS) reached an agreement to jointly develop and promote the investigational ADC MORAb-202 targeting the folate receptor α (FRα). The small molecule toxin used in this ADC is eribulin. Eribulin is a tubulin polymerization inhibitor. It is a synthetic analog of halichondrin B. Halichondrin B is a substance discovered from black sponges growing along the coast of Japan. It was first approved by the US FDA in 2010 for the treatment of metastatic breast cancer and can effectively cure tumors. This drug is the only single-agent chemotherapy drug available. In addition to its very significant therapeutic effect on metastatic breast cancer, eribulin can be used for multiple indications, so there is huge room for its late-stage development, and it is a drug with great ADC development value.

One of the most common linking sites on antibodies used for conjugation in ADC is lysine, and its ε-amino group can react with the activated carboxyl group of the linker to form an amide bond. However, such amide bonds are prone to hydrolysis under the action of enzymes in the body, causing the bioactive molecules and antibodies to fall off before they reach the target cells. This results in the loss of ADC targeting and increased toxicity. Another common linking site is a cysteine residue. The thiol groups of antibody cysteine all exist in the form of disulfide bonds. By opening the disulfide bonds in the antibody, multiple free thiol groups as conjugating sites can be provided. To conjugate the thiol group in the antibody, one method is to have a Michael addition reaction between the free thiol group in the antibody and maleimide, or it can also be implemented by two Michael addition reactions between a specific substrate and the free thiol group in the antibody to form a sulfur bridge having a unique structure. However, as reported, the Michael reaction turns in reverse Michael addition in the systemic circulation, resulting in premature shedding of toxins and toxic reactions.

As stated above, the small molecule toxin eribulin is at least 10 times more toxic than DNA-damaging toxoids such as camptothecin derivatives. Therefore, in development of an ADC based on eribulin, it is even more important to consider its safety. At present, there are few modifications and innovations based on the reactive groups of ADC antibody linking sites, and especially for the development of ADC linkers to eribulin, there is still a lot of room. That is, there is still a need in this field to improve the reactive groups of the linking sites to obtain ADCs with smaller off-target effects and higher effectiveness.

### SUMMARY

The present disclosure provides an antibody-drug conjugate (ADC) with the effect of inhibiting tumor cells. The ADC has an improved linker moiety, which has an antibody conjugating reaction group that is not easily cleaved, and a more specific enzyme cleavage site than an acid-base sensitive linker, thereby increasing the blood flow stability of the ADC and reducing off-target effects. At the same time, it has the ability to quickly and specifically release small molecule toxins after reaching the tumor site and has high conjugating efficiency and high drug loading capacity (the DAR is between 5 and 8). Compared with currently marketed or clinically popular ADCs, it has better therapeutic effects and treatment windows in some tumor models.

In a first aspect of the present disclosure, provided is a compound of formula (I), a pharmaceutically acceptable salt thereof, a stereoisomer thereof, or a solvate of the compound, the pharmaceutically acceptable salt of the compound or the stereoisomer of the compound,

L¹-L2-L³-L⁴-D (I)

wherein L¹ is 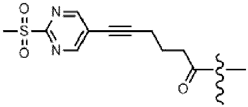 , L² is a single bond or -NH-R¹-(CO)-,
orL¹-L² is 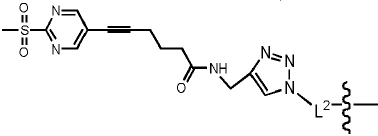 , wherein L² is -R¹-(CO)-;
R¹ is selected from -(CH₂)ₐ-, -(CH₂CH₂O)_{b}-(CH₂)_{c}-, and -CH₂CH₂-(OCH₂CH₂)_{b}-NH-(CO)-CH₂-O-CH₂-;
L³ is a peptide residue, selected from the group consisting of glycine-glycine-phenylalanine-glycine (GGFG), glutamic acid-valine-citrulline (EVC), valine-citrulline (VC), aspartic acid-valine-citrulline (DVC), glutamic acid-glycine-glycine-phenylalanine-glycine (EGGFG), and aspartic acid-glycine-glycine-phenylalanine-glycine (DGGFG), the structural formulas are respectively:

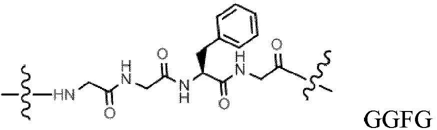

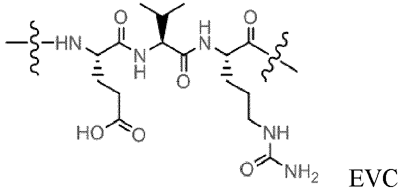

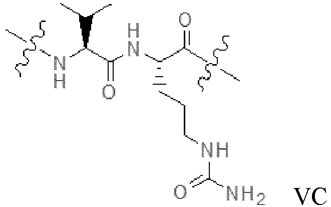

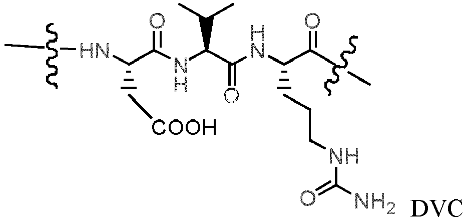

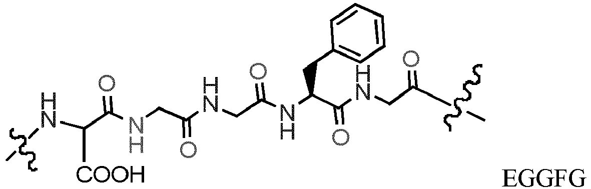

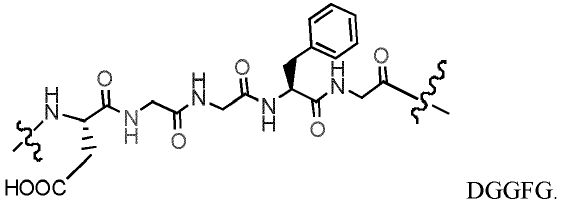

L⁴ is selected from the group consisting of a single bond, -NH-CH₂-, and 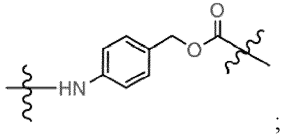
D is a drug linked to L⁴ via a chemical bond, and the drug is selected from the group consisting of eribulin or its derivative, camptothecin drugs, auristatins (such as monomethyl auristatin E(MMAE), and monomethyl auristatin F(MMAF));
a is selected from an integer between 1 and 6, such as, 1, 2, 3, 4, 5, and 6;
b is independently selected from an integer between 1 and 20, such as 1, 2, 3, 4, 5, 6, 7, 8, 9, 10, 11, 12, 13, 14, 15, 16, 17, 18, 19, and 20, for example, selected from an integer between 1 and 8;
c is 1 or 2.

In some embodiments, L³ is selected from GGFG,VEC, and VC.

In some embodiments, R¹ is -(CH₂)ₐ-. In some embodiments, R¹ is -(CH₂CH₂O)_{b}-(CH₂)_{c}-. In some embodiments, R¹is -CH₂CH₂-(OCH₂CH₂)_{b}-NH-(CO)-CH₂-O-CH₂-.

In some embodiments, L² is a single bond. In some embodiments, L² is -NH-R¹-(CO)-.

In some embodiments, L³ is GGFG. In some embodiments, L³ is VEC. In some embodiments, L³ is VC.

In some embodiments, L⁴ is -NH-CH₂-. In some embodiments, L⁴ is a single bond. In some embodiments, L⁴ is

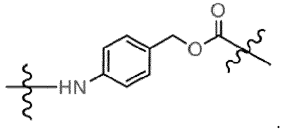

In some embodiments, L¹ is 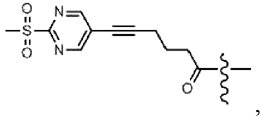 L² is -NH-R¹-(CO)-, R¹ is -(CH₂)ₐ-, and a is 2, 3, 4, 5 or 6.

In some embodiments, L¹ is 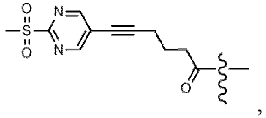 L² is -NH-R¹-(CO)-, R¹ is - (CH₂CH₂O)_{b}-(CH₂)_{c}-, b is 2, 3, 4, 5, 6

In some embodiments, L¹ is 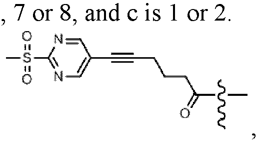 L² is a single bond.

In some embodiments, L¹ is 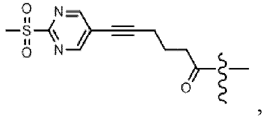 L² is -NH-R¹-(CO)-, R¹ is - CH₂CH₂-(OCH₂CH₂)_{b}-NH-(CO)-CH₂-O

In some embodiments, L¹-L² is 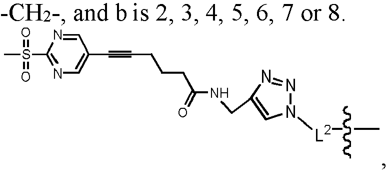 wherein L² is -R¹-(CO)-, R¹ is -CH₂CH₂-(OCH₂CH₂)_{b}-NH-(CO)-CH₂-O-CH₂-; In some embodiments, b is 2, 3, 4, 5, 6, 7 or 8.

In some embodiments, L¹ is 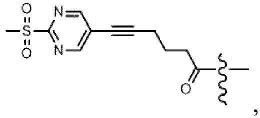 L² is -NH-R¹-(CO)-, R¹ is -(CH₂)ₐ-, a is 2, 3, 4, 5 or 6, and L³ is GGFG.

In some embodiments, L¹ is 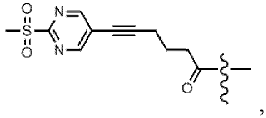 L² is -NH-R¹-(CO)-, R¹ is - (CH₂CH₂O)_{b}-(CH₂)_{c}-, b is 2, 3, 4, 5, 6, 7 or 8, cis 1 or 2, and L³ is GGFG or EVC.

In some embodiments, L¹ is 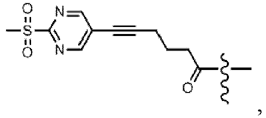 L² is a single bond, and L³ is GGFG.

In some embodiments, L¹ is 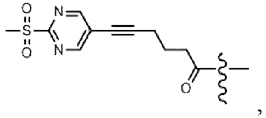 L² is -NH-R¹-(CO)-, R¹ is - CH₂CH₂-(OCH₂CH₂)_{b}-NH-(CO)-CH₂-O-CH₂-, b is 2, 3, 4, 5, 6, 7 or 8, and L³ is EVC.

In certain embodiments, L¹-L² is 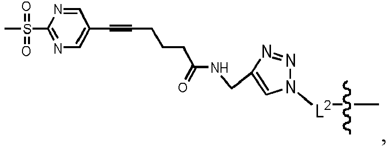 wherein L² is - R¹-(CO)-, R¹ is -CH₂CH₂-(OCH₂CH₂)_{b}-NH-(CO)-CH₂-O-CH₂-, b is 2, 3, 4, 5, 6, 7 or 8, and L³ is EVC.

In some embodiments, L¹ is 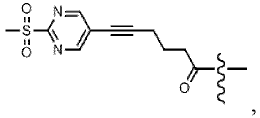 L² is a single bond, and L³ is VC or GGFG.

In some embodiments, L² is selected from the group consisting of a single bond,

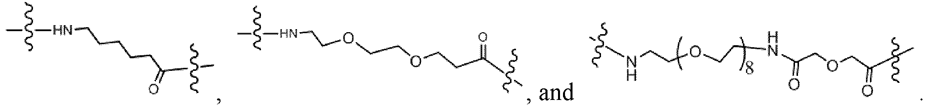

In some embodiments, L¹-L² is

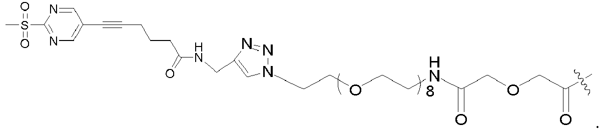

In some embodiments, L³ is GGFG, and L⁴ is a single bond.

In some embodiments, L³ is GGFG, and L⁴ is -NH-CH₂-.

In some embodiments, L³ is EVC, and L⁴ is

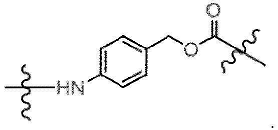

In some embodiments, L³ is GGFG, and L⁴ is

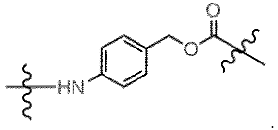

In some embodiments, D is eribulin or its derivative. In some embodiments, D is selected from the group consisting of 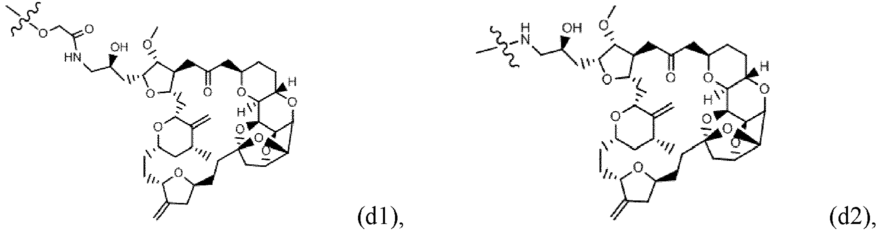 and

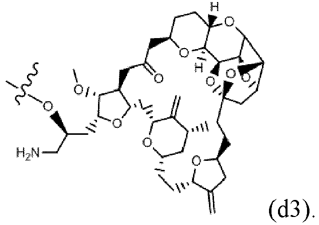

In some embodiments, L⁴ is -NH-CH₂-, and D is d1.

In some embodiments, L⁴ is -NH-CH₂-, and D is d2.

In some embodiments, L⁴ is -NH-CH₂-, and D is d3.

In some embodiments, L⁴ is a single bond, and D is d2.

In some embodiments, L⁴ is 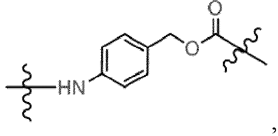 and D is d2.

In some embodiments, the compound of formula (I) is selected from:

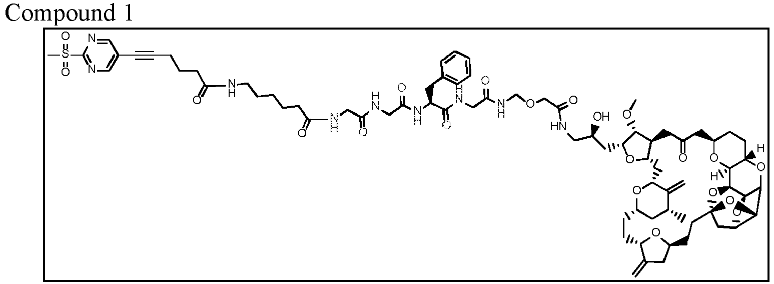

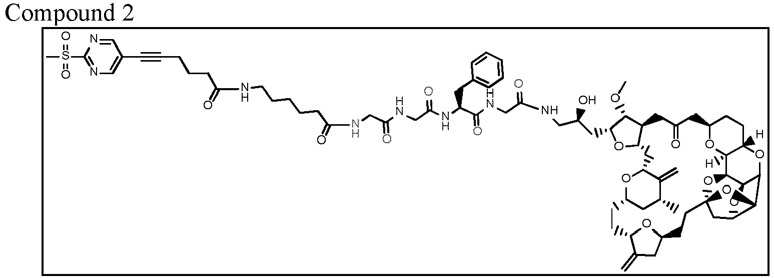

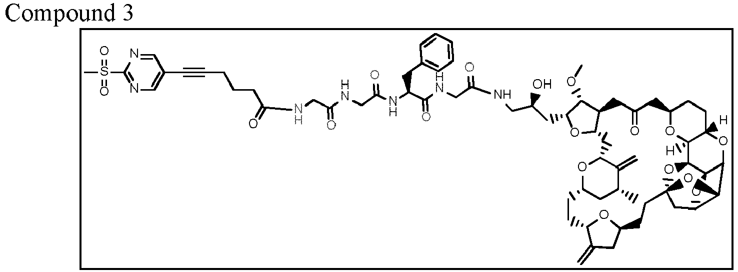

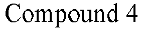

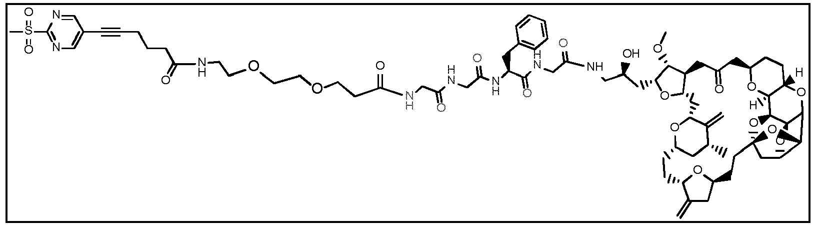

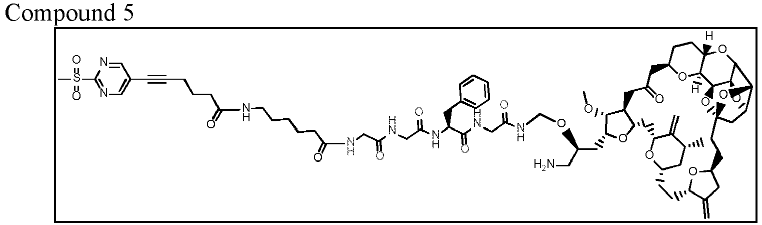

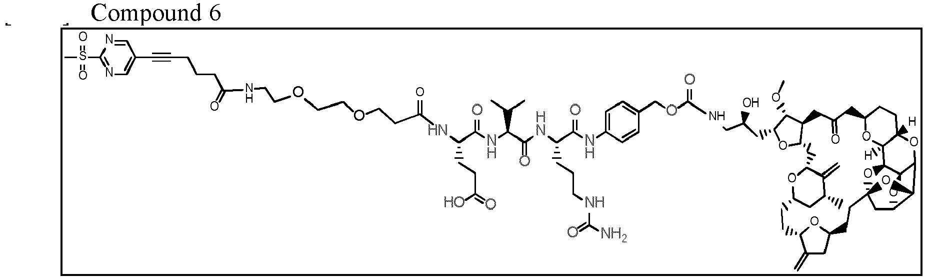

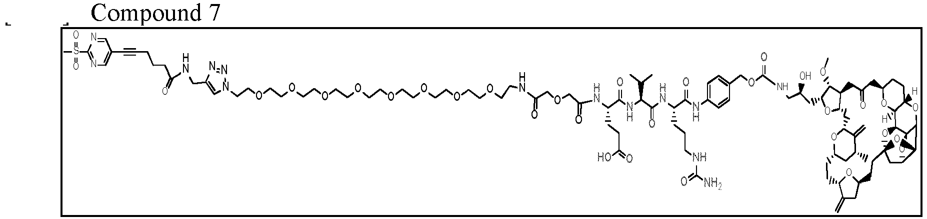

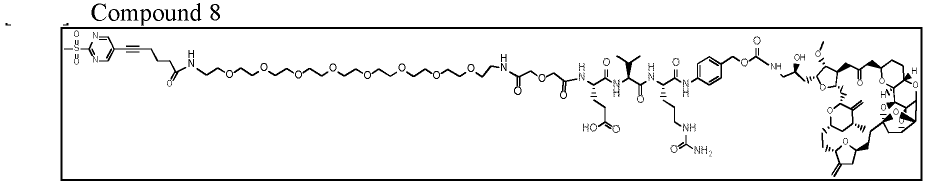

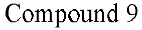

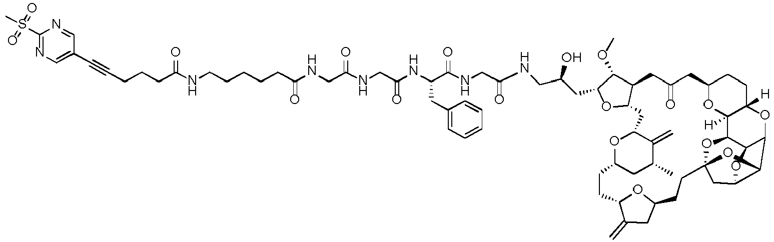

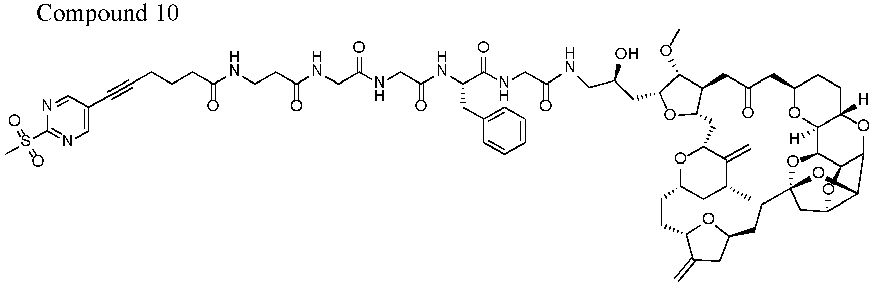

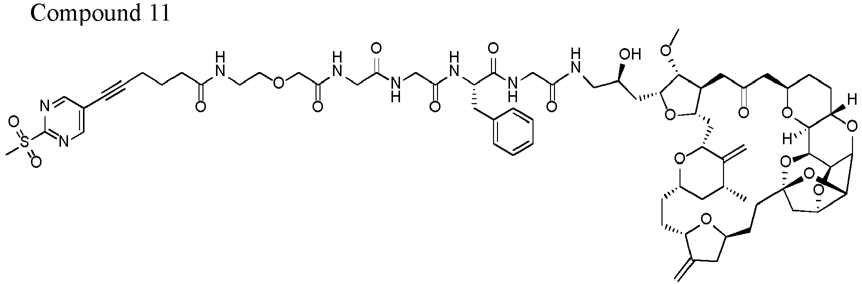

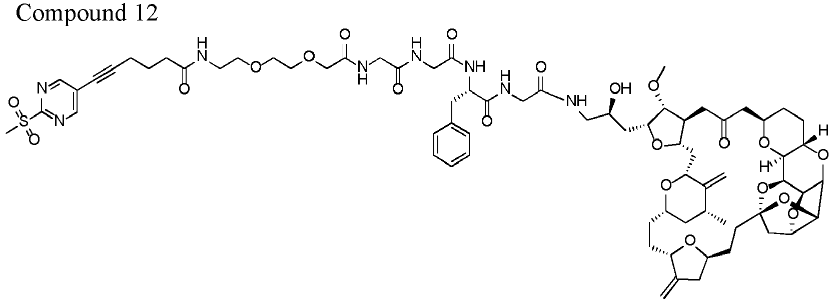

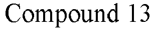

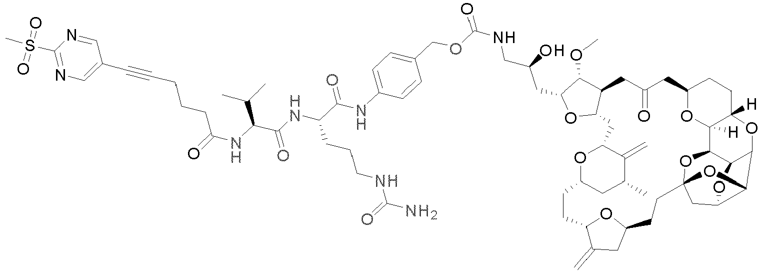

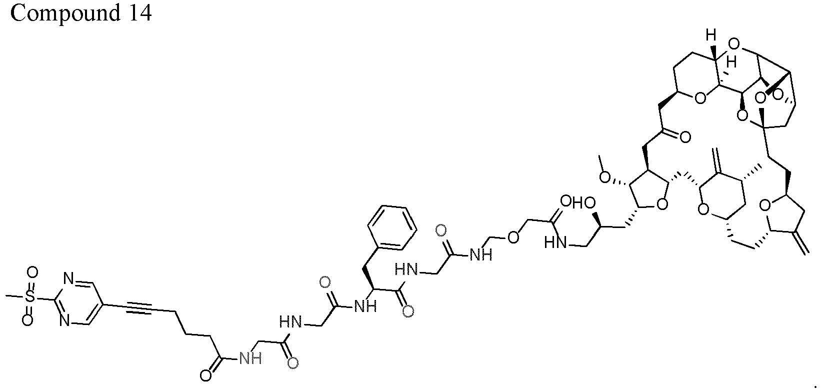

The compound of formula (I) can be used as a connector-drug intermediate compound for the synthesis of an antibody-drug conjugate. Therefore, in the second aspect of the present disclosure, provided is an antibody-drug conjugate, a pharmaceutically acceptable salt thereof, a stereoisomer thereof, or a solvate of the conjugate, the pharmaceutically acceptable salt of the conjugate or the stereoisomer of the conjugate, which can be formed by linking a targeting moiety to the compound of formula (I) via a thioether linkage.

In some embodiments, the antibody-drug conjugate has a structure of formula (II)

Ab-(S-L¹-L²-L³-L⁴-D)ₚ (II)

wherein Ab is a targeting moiety selected from an antibody, an antibody fragment, or an antibody-based molecule or compound;
-S- is a thioether linkage,
L¹, L², L³, L⁴, and D are defined as above,
p is an integer between 1 and 20, such as 1, 2, 3, 4, 5, 6, 7, 8, 9, 10, 11, 12, 13, 14, 15, 16, 17, 18, 19, and 20, for example, selected from an integer between 1 and 8.

In some embodiments, the Ab targeting moiety is an antibody, an antibody fragment, a bispecific or other multivalent antibody, or other antibody-based molecule or compound. The antibody may be of various isotypes, preferably human IgGl, IgG2, IgG3 or IgG4, and more preferably comprise human IgGl hinges and constant region sequences. The antibody or a fragment thereof may be a chimeric human-mouse, chimeric human-primate, humanized (human framework and mouse hypervariable (CDR) regions) or fully human antibody and variants thereof, such as half-IgG4 antibodies (referred to as "monoclonal antibodies") as described by van der Neut Kolfschoten et al. (Science 2007; 317:1554-1557). More preferably, the antibody or the fragment thereof can be designed or selected to comprise human constant region sequences belonging to a specific allotype, which can result in reduced immunogenicity when the antibody or an immunoconjugate thereof is administered to a human subject. A preferred allotype for administration includes a non-G1m1 allotype (nG1m1), such as G1m3, G1m3,1, G1m3,2 or G1m3,1,2. More preferably, the allotype is selected from the group consisting of allotypes of nG1m1, G1m3, nG1m1,2, and Km3.

A suitable antibody may bind to any disease-associated antigen known in the art. When the disease state is a cancer, for example, many antigens expressed by or otherwise associated with tumor cells are known in the art, including but not limited to carbonic anhydrase IX, alpha-fetoprotein (AFP), α-actinin-4, A3, antigens specific for A33 antibody, ART-4, B7, Ba 733, BAGE, BrE3 antigen, CA125, CAMEL, CAP-1, CASP-8/m, CCL19, CCL21, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD44, CD45, CD46, CD52, CD54, CD55, CD59, CD64, CD66a-e, CD67, CD70, CD70L, CD74, CD79a, CD80, CD83, CD95, CD126, CD132, CD133, CD138, CD147, CD154, CDC27, CDK-4/m, CDKN2A, CTLA-4, CXCR4, CXCR7, CXCL12, HIF-1α, colon-specific antigen p (CSAp), CEA (CEACAM5), CEACAM6, c-Met, DAM, EGFR, EGFRvIII, EGP-1 (Trop-2), EGP-2, ELF2-M, Ep-CAM, claudin 18.2, ROR1, ROR2, fibroblast growth factors (FGFs), Flt-1, Flt-3, folate receptors, G250 antigen, GAGE, gp100, GRO-β, HLA-DR, HM1.24, human chorionic gonadotropin (HCG) and its subunits, HER2/neu, HMGB-1, hypoxia inducible factor (HIF-1), HSP70-2M, HST-2, Ia, IGF-1R, IFN-γ, IFN-α, IFN-β, IFN-λ, IL-4R, IL-6R, IL-13R, IL-15R, IL-17R, IL-18R, IL-2, IL-6, IL-8, IL-12, IL-15, IL-17, IL-18, IL-23, IL-25, insulin-like growth factor 1(IGF-1), IGF-1R, KS1-4, Le-Y, LDR/FUT, macrophage migration inhibitory factors (MIFs), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5ac, MUC13, MUC16, MUM-1/2, MUM-3, NCA66, NCA95, NCA90, pancreatic cancer mucin, PD-1 receptor, PD-L1 receptor, placental growth factors, p53, PLAGL2, prostatic acid phosphatase, PSA, PRAME, PSMA, PlGF, ILGF, ILGF-1R, IL-6, IL-25, RS5, RANTES, T101, SAGE, S100, survivin, survivin-2B, TAC, TAG-72, tenascin, TRAIL receptor, TNF-α, Tn antigen, Thomson-Friedenreich antigen, tumor necrosis antigens, VEGFR, ED-B fibronectin, WT-1, 17-1A antigen, complement factors C3, C3a, C3b, C5a, C5, angiogenesis markers, bcl-2, bcl-6, Kras, oncogene markers and oncogene products (see, e.g., Sensi et al., Clin Cancer Res2006, 12:5023-32; Parmiani et al., J Immunol 2007, 178:1975-79; Novellino et al. Cancer Immunol Immunother 2005, 54:187-207). Preferably, the antibody binds to her2, her3, claudin 18.2, ROR-1, dll-3, muc1, muc-17, or EGP-1(Trop-2).

Exemplary antibodies that may be used include, but are not limited to, hR1 (anti-IGF-1R, U.S. Pat. 13/688,812), hPAM4 (antimucin, U.S. Pat. 7,282,567), hA20 (anti-CD20, U.S. Pat. 7,151,164), hA19 (anti-CD19, U.S. Pat. 7,109,304), hIMMU31 (anti-AFP, U.S. Pat. 7,300,655), hLL1 (anti-CD74, U.S. Pat. 7,312,318), hLL2 (anti-CD22, U.S. Pat. 5,789,554), hMu-9 (anti-CSAp, U.S. Pat. 7,387,772), hL243 (anti-HLA-DR, U.S. Pat. 7,612,180), hMN-14 (anti-CEACAM5, U.S. Pat. 6,676,924), hMN-15 (anti-CEACAM6, U.S. Pat. 8,287,865), hRS7 (anti-EGP-1, U.S. Pat. 7,238,785), hMN-3 (anti-CEACAM6, U.S. Pat. 7,541,440), Ab124 and Ab125 (anti-CXCR4, U.S. Pat. 7,138,496). The Examples of all the cited patents or applications are incorporated herein by reference. More preferably, the antibody is IMMU-31 (anti-AFP), hRS7 (anti-Trop-2), hMN-14 (anti-CEACAM5), hMN-3 (anti-CEACAM6), hMN-15 (anti-CEACAM6), hLL1 (anti-CD74), hLL2 (anti-CD22), hL243 or IMMU-114 (anti-HLA-DR), hA19 (anti-CD19), or hA20 (anti-CD20). As used herein, the terms "epratuzumab" and "hLL2" are interchangeable, the same to the terms "veltuzumab" and "hA20", hL243g4P, hL243y4P and IMMU-114. In a most preferred embodiment, the antibody is an anti-Trop-2 antibody, such as hRS7; and anti-ROR1 antibodies, such as 99961(CN patent No. 104662044).

Suitable alternative antibodies include, but are not limited to, abciximab (anti-glycoprotein Ilb/IIIa), alemtuzumab (anti-CD52), bevacizumab (anti-VEGF), cetuximab (anti-EGFR), gemtuzumab (anti-CD33), ibritumomab (anti-CD20), panitumumab (anti-EGFR), rituximab (anti-CD20), tositumomab (anti-CD20), trastuzumab (anti-ErbB2), lambrolizumab (anti-PD1 receptor), atezolizumab (anti-PD-L1), MEDI4736 (anti-PD-L1), nivolumab (anti-PD-1 receptor), ipilimumab (anti-CTLA-4), abagovomab (anti-CA-125), adecatumumab (anti-EpCAM), atlizumab (anti-IL-6 receptor), benralizumab (anti-CD125), obinutuzumab (GA101, anti-CD20), CC49 (anti-TAG-72), AB-PG1-XG1-026 (anti-PSMA, US Patent Application 11/983,372, deposit No. ATCC PTA-4405 and PTA-4406), D2/B (anti-PSMA, WO 2009/130575), tocilizumab (anti-IL-6 receptor), basiliximab (anti-CD25), daclizumab (anti-CD25), efalizumab (anti-CD11a), GA101 (anti-CD20; GlycartRoche), muromonab-CD3 (anti-CD3 receptor), natalizumab (anti-α4 integrin), omalizumab (anti-IgE); anti-TNF-α antibody, such as CDP571 (Ofei et al., 2011, Diabetes 45:881-85), MTNFAI, M2TNFAI, M3TNFAI, M3TNFABI, M302B, M303 (ThermoScientific, Rockford, IL), infliximab (Centocor, Malvern, PA), certolizumab pegol (UCB, Brussels, Belgium), anti-CD40L (UCB, Brussels, Belgium), adalimumab (Abbott, Abbott Park, IL), Benlysta (Human Genome Sciences); antibodies for treatment of Alzheimer' s disease, such as Alz 50 (Ksiezak-Reding et al., 1987, J Biol Chem 263:7943-47), gantenerumab, solanezumab and infliximab; antifibrin antibodies such as 59D8, T2G1s, MH1; anti-CD38 antibodies, such as MOR03087 (MorphoSys AG), MOR202 (Celgene), HuMax-CD38(Genmab) or daratumumab (Johnson & Johnson); (anti-HIV antibodies, such as P4/D10 (U.S. Pat. 8,333,971), Ab75, Ab76, Ab77 (Paulik et al., 1999, Biochem Pharmacol 58:1781-90), and anti-HIV antibodies described and marketed by Polymun (Vienna, Austria), also described in U.S. Pat. 5,831,034, U.S. Pat. 5,911,989 and Vcelar et al., AIDS 2007; 21(16):2161-2170 and Joos et al., Antimicrob. Agents Chemother. 2006; 50(5):1773-9, which are all incorporated herein by reference.

In some embodiments, the targeting moiety is a hRS7 antibody. The amino acid sequences of the light chain and heavy chain in the hRS7 antibody are as shown in SEQ ID NO:1 and SEQ ID NO:2 respectively, and their corresponding nucleotide sequences are as shown in SEQ ID NO: 3 and SEQ ID NO:4, respectively.

In some embodiments, the drug moiety conjugated to the subject antibody is selected from eribulin or a derivative thereof, such as a mesylate salt of eribulin. As used herein, the term "eribulin" refers to a synthetic analog of halichondrin B, a macrocyclic compound originally isolated from the sponge Halichondria okadais. Eribulin is a microtubule kinetic inhibitor and is believed to bind to tubulin and cause cell cycle arrest in the G2/M phase by inhibiting mitotic spindle components. The term "eribulin mesylate" refers to the mesylate salt of eribulin, which is sold under the tradename Halaven^{™}.

The drug moiety may also be a water-soluble camptothecin compound or a derivative thereof, such as Exatecan, Irinotecan, Chimmitecan, dxd, Gimatecan, SN-38 or CPT-11. Extensive clinical data are available involving the pharmacology of CPT-11 and its conversion to active SN-38 in vivo (Iyer and Ratain, Cancer Chemother Pharmacol. 42: S31-43 (1998); Mathijssen et al., Clin Cancer Res. 7: 2182-2194 (2002); Rivory, Ann NY Acad Sci. 922: 205-215, 2000)). The active form of SN-38 is approximately 2 to 3 orders of magnitude more potent than CPT-11.

The drug moiety may also be selected as auristatins (such as monomethyl auristatin E (MMAE), monomethyl auristatin F (MMAF)).

In some embodiments, the antibody or the fragment thereof is linked to at least one chemotherapeutic moiety, for example, 1 to 5 (e.g., 1, 2, 3, 4, or 5) drug moieties, 5 to 8 (e.g., 5, 6, 7, or 8) drug moieties or 8 to 12 (8, 9, 10, 11, or 12) drug moieties.

In a third aspect, the present disclosure provides a preparation method of the compound, pharmaceutically acceptable salt, stereoisomer or solvate described in the first aspect and the antibody-drug conjugate, pharmaceutically acceptable salt, stereoisomer or solvate described in the second aspect.

The preparation method of the compound of formula (I):
If L² is a single bond and L⁴ is 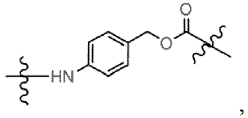 the method comprises the following steps: 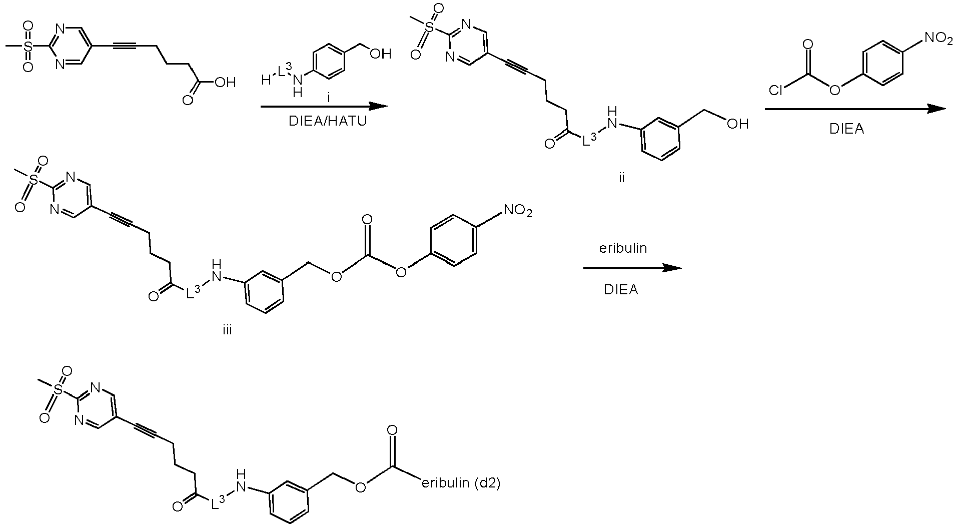
(1) allowing 6-(2-(methylsulfonyl)pyrimidine)-5-hexynoic acid and compound i to have a condensation reaction in the presence of DIEA and HATU to generate compound ii;
(2) allowing compound ii and p-nitrophenyl chloroformate to have a condensation reaction in the presence of DIEA to generate compound iii; and
(3) allowing compound iii and eribulin to have a condensation reaction in the presence of DIEA to generate the compound of formula (I) (D is d2).

If L² is a single bond and L⁴ is -NH-CH₂-, the method comprises the following steps: 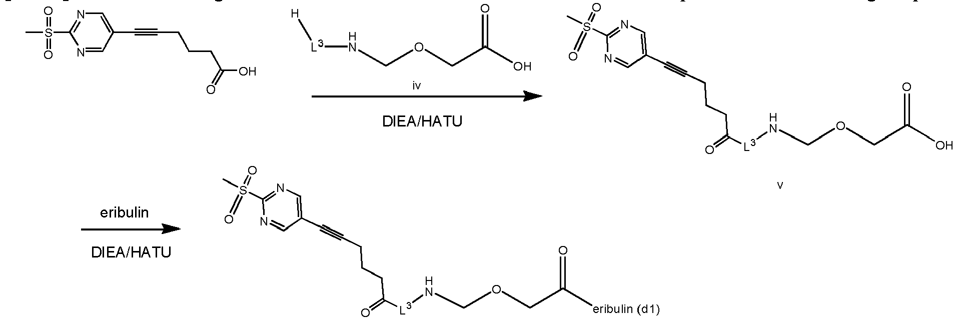
(1) allowing 6-(2-(methylsulfonyl)pyrimidine)-5-hexynoic acid and compound IV to have a condensation reaction in the presence of DIEA and HATU to generate compound V; and
(2) allowing compound V and eribulin to have a condensation reaction in the presence of DIEA and HATU to generate the compound of formula (I) (D is d2).

If L² is a single bond and L⁴ is a single bond, the method comprises the following steps: 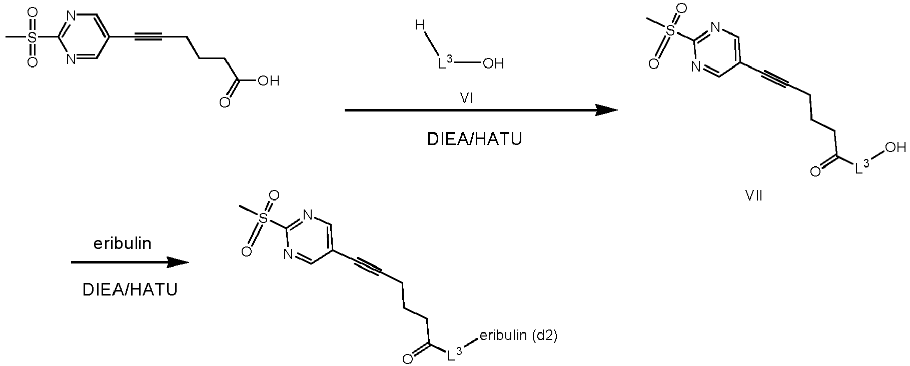
(1) allowing 6-(2-(methylsulfonyl) pyrimidine)-5-hexynoic acid and compound VI to have a condensation reaction in the presence of DIEA and HATU to generate compound VII; and
(2) allowing compound VII and eribulin to have a condensation reaction in the presence of DIEA and HATU to generate the compound of formula (I) (D is d2).

The above three synthesis methods can be applied to the synthesis of compounds in which L² is a single bond. If L² is not a single bond, L¹-L² may be prepared first, and then synthesis procedures proceed according to the above three methods. L¹-L² may be generated through a condensation reaction between the carboxyl group and the amino group to form an amide bond, or L¹-L² may be generated through a click reaction of the azide-alkynyl group.

The above three synthesis methods are exemplarily described below.

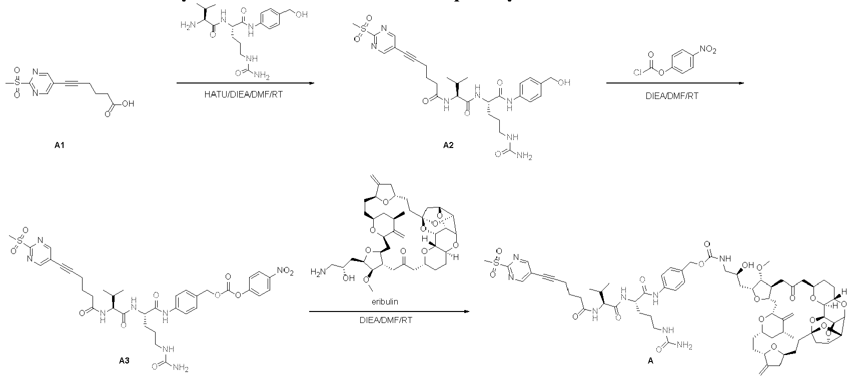

6-(2-(methylsulfonyl)pyrimidine)-5-hexynoic acid and HATU (1.5 equivalents) were placed in a reaction bottle and dissolved with an appropriate amount of DMF, and then DIEA (2.0 equivalents) was added to react for about half an hour. Then, compound VC-PAB-OH (1.0 equivalent) was added to react for another 3 h. The crude product was purified to give the product A2 of interest. The linker A2 and p-nitrophenyl chloroformate (1.5 equivalents) were completely dissolved in an appropriate amount of DMF solvent. Then, 2 equivalents of DIEA were added to react for about 3 h. The crude product was purified to give the product A3 of interest. A3 and an equal molar ratio of eribulin were placed in a reaction bottle and dissolved with an appropriate amount of DMF. Then, DIEA (2.0 equivalents) was added to react for 3 h. The crude product was purified to give the product A of interest. Intermediate E in Example 3 was synthesized according to this method.

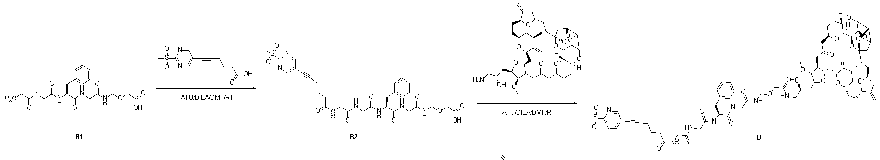

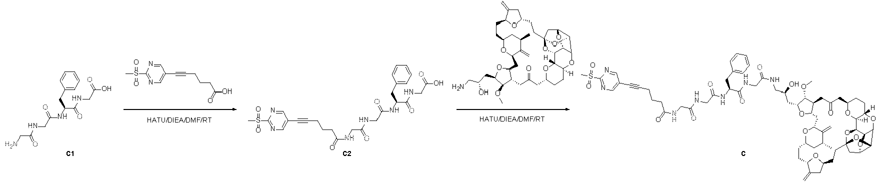

6-(2-(methylsulfonyl)pyrimidine)-5-hexynoic acid and an equal molar ratio of HATU were placed in a reaction bottle and dissolved with an appropriate amount of DMF, and then DIEA (2.0 equivalents) was added to react for about 2 h. Then, compound B1 or C1 (1.0 equivalent) was added to react for another 3 h. The crude product was purified to give the product B2 or C2 of interest. B2 or C2 and HATU (1.5 equivalents) were added to the reaction bottle and dissolved in an appropriate amount of DMF. Then, DIEA (2.0 equivalents) was added to react for about half an hour, and the compound eribulin (1.0 equivalent) was added to react for another 3 h. The crude product was purified to give the product B or C of interest. Intermediate D in Example 2 and Intermediate F in Example 4 were synthesized according to this method.

### Preparation method of antibody-drug conjugate represented by formula (II)

In the present invention, an antibody-drug conjugate in which an antibody and a connector structure are linked via a thioether may be produced by the following method.

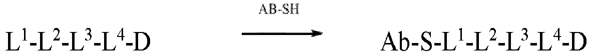

That is, the compound, pharmaceutically acceptable salt, stereoisomer or solvate described in the first aspect as a connector-drug intermediate reacts with a targeting moiety (e.g., an antibody) such that the connector-drug intermediate is linked to the targeting moiety via a thioether linkage formed by the disulfide bond portion of the hinge region in the targeting moiety, thereby preparing the antibody-drug conjugate of formula (II).

L¹, L², L³, L⁴, D, Ab, and S are defined as described in the specification of the present invention.

For convenience of explanation, the antibody-drug conjugate of formula (II) is described as a structure in which one structural moiety from the drug to the end of the connector is linked to one antibody. However, in fact, in many cases, multiple such structural moieties are linked to one antibody molecule. For example, as described above, 2 to 8, preferably 4 to 8, more preferably 6 to 8 connector-drug intermediate compounds are linked to one antibody molecule. This case is also applicable to the following description of the production method. In fact, as described above, in the present invention, the average number of connector-drug structures linked to each antibody molecule is expressed as the average number of drug moieties linked.

That is, as shown above, by reacting the connector-drug intermediate compound of the present invention with the antibody Ab-SH having a thiol group, the antibody-drug conjugate of formula (II) can be produced.

Specifically, as a reducing agent, 1 to 7 molar equivalents of TCEP per hinge disulfide bond within the antibody is used to react with the antibody in a buffer containing the chelating agent EDTA, thereby obtaining an antibody (AB-SH) carrying a thiol group by partially or completely reducing the disulfide in the hinge region of the antibody. Examples of chelating agent include ethylenediaminetetraacetic acid (EDTA), diethylenetriaminepentaacetic acid (DTPA), and the like. They can be used at concentrations of 1 mM~20 mM. Sodium phosphate, sodium borate, sodium acetate solution or the like may be used as a buffer. In a specific example, by reacting the antibody with TCEP at a temperature of 4 °C~37 °C for 1~4 h, an antibody that is partially or completely reduced and antibody AB-SH having a thiol group can be obtained.

For each antibody Ab-SH having a thiol group, 2 to 20 molar equivalents of the compound of formula (I) can be used to produce an antibody-drug conjugate (II) in which 2 to 8 drug moieties are linked to one antibody molecule. Specifically, a solution in which the compound of the formula (I) is dissolved is added to a buffer containing the antibody Ab-SH having a thiol group to have a reaction. Here, sodium acetate solution, sodium phosphate, sodium borate, or the like may be used as a buffer. The pH during the reaction is between 5 and 9, and more preferably around pH7 during the reaction. An organic solvent such as dimethyl sulfoxide (DMSO), dimethylformamide (DMF), dimethylacetamide (DMA), and N-methyl-2-pyridone (NMP) can be used as a solvent for dissolving the compound. An organic solvent solution in which the compound of formula (I) is dissolved is added to a buffer containing the antibody Ab-SH having a thiol group at 1 to 20% v/v to have a reaction. The reaction is carried out at 0~37 °C, more preferably at 10-25 °C, for 0.5-2 h. The reaction can be terminated by deactivating the reactivity of the unreacted compound of formula (I) using a mercaptan-containing reagent. The mercaptan-containing reagent is, for example, cysteine or N-acetyl-L-cysteine (NAC). More specifically, the reaction is terminated by adding 1 to 2 molar equivalents of cysteine relative to the compound of formula (I) used and incubating at room temperature for 10-30 min.

For the produced antibody-drug conjugate (II), the following common procedures can be implemented to perform operations such as concentration, buffer exchange, and purification.

### Common procedure A: Concentration of a solution of an antibody or an antibody-drug conjugate in water

A solution of an antibody or an antibody-drug conjugate is placed in a container and then centrifuged on a centrifuge (e.g., at 2000 G~3800 G for 5-20 min) to concentrate the solution of the antibody or the antibody-drug conjugate.

### Common procedure B: Determination of antibody concentration

Using a UV determinator, antibody concentration is measured according to the manufacturer's instructions.

In this case, the 280 nm absorbance coefficient (1.3 mLmg⁻¹ cm⁻¹-1.8 mLmg⁻¹ cm⁻¹) that varies depending on the antibody is used.

### Common procedure C-1: Buffer exchange for an antibody

According to the manufacturer's instructions, a phosphate buffer (e.g., PBS) containing NaCl (e.g., 137 mM) and ethylenediaminetetraacetic acid (EDTA) (e.g., 5 mM) (also referred to herein as PBS/EDTA) is used. A PD-10 column using the Sephadex G-25 carrier is equilibrated. For one PD-10 column, 1 mL of a solution of an antibody in water is loaded. Then, the fraction (3.5 mL) eluted with 2 mL of PBS/EDTA is separated. The fraction is concentrated by common procedure A, the antibody concentration is measured by common procedure B, and then the antibody concentration is adjusted, using PBS/EDTA, to 10 mg/mL.

### Common procedure C-2: Buffer exchange for the antibody

According to the manufacturer's instructions, a phosphate buffer containing NaCl (e.g., 50 mM) and EDTA (e.g., 2 mM) (e.g., 50 mM, pH 6.5, also referred to herein as PBS6.5/EDTA) is used. A PD-10 column using the Sephadex G-25 carrier is equilibrated. For one PD-10 column, 1 mL of a solution of an antibody in water is loaded. Then, the fraction (3.5 mL) eluted with 2 mL of PBS6.5/EDTAis separated and obtained. The fraction is concentrated by common procedure A, the antibody concentration is measured by common procedure B, and then the antibody concentration is adjusted, using PBS6.5/EDTA, to 5 mg/mL.

### Common operation D-1: Purification of an antibody-drug conjugate

The PD-10 column is equilibrated using any one of a commercially available phosphate buffer (e.g., PBS7.4), a NaCl (e.g., 137 mM)-containing sodium phosphate buffer (e.g., 10 mM, pH6.5; also referred to herein as PBS6.5), or a sorbitol (e.g., 5%)-containing acetate buffer (e.g., 10 mM, pH 5.5; also referred to herein as ABS) or MES 25 mM pH 6.5, or His 10 mM pH 5.5. The column is loaded with a reaction solution of an antibody-drug conjugate in water (e.g., about 1 mL), and an antibody fraction is separated by elution with a buffer in an amount specified by the manufacturer, thereby obtaining an antibody-drug conjugate in which unlinked drug connectors and low molecular compounds (such as tris(2-carboxyethyl)phosphine hydrochloride (TCEP), cysteine, and dimethyl sulfoxide) are removed.

### Common operation D-2: Purification of an antibody-drug conjugate

An AKTA column (packing: sephadex G 25) is equilibrated using any one of a phosphate buffer (e.g., PBS7.4), a NaCl (e.g., 137 mM)-containing sodium phosphate buffer (e.g., 10 mM, pH 6.5; also referred to herein as PBS6.5), or a sorbitol (e.g., 5%)-containing acetate buffer (e.g., 10 mM, pH 5.5; also referred to herein as ABS) or MES 25 mM pH 6.5, or His 10 mM pH 5.5. A sample injector is loaded with the reaction solution of an antibody-drug conjugate in water (e.g., about 2 mL), and an antibody fraction is separated by elution with a buffer in an amount specified by the manufacturer, thereby obtaining an antibody-drug conjugate in which unlinked drug connectors and low molecular compounds (such as tris(2-carboxyethyl)phosphine hydrochloride (TCEP), cysteine, and dimethyl sulfoxide) are removed.

In a fourth aspect, the present disclosure provides a pharmaceutical composition, comprising the compound, pharmaceutically acceptable salt, stereoisomer or solvate described in the first aspect or the antibody-drug conjugate, pharmaceutically acceptable salt, stereoisomer or solvate described in the second aspect, and one or more pharmaceutical excipients, such as carriers and/or excipients.

The pharmaceutical composition may be made into any pharmaceutically acceptable dosage form. The pharmaceutical composition may also be administrated to individuals in need of such treatment in any suitable way of administration, such as oral, parenteral, rectal or pulmonary administration. In the case of oral administration, the pharmaceutical composition may be made into conventional solid preparations, such as tablets, capsules, pills, granules, etc. It may also be made into oral liquid preparations, such as oral solutions and oral suspensions, and syrups. In the case of oral preparations, suitable fillers, binders, disintegrants, lubricants, etc. may be added. In the case of parenteral administration, the pharmaceutical composition may be made into injection preparations, including injection solutions, sterile powders for injection, and concentrated solutions for injection. In the case of injection preparations, they may be produced by a conventional method in the current pharmaceutical field. In the case of preparation of injection preparations, additives may be not added, or appropriate additives may be added according to the properties of the medicament. In the case of rectal administration, the pharmaceutical composition may be made into suppositories and the like. In the case of pulmonary administration, the pharmaceutical composition may be made into an inhalant or a spray.

In a fifth aspect, the present disclosure provides a use of the compound, pharmaceutically acceptable salt, stereoisomer or solvate described in the first aspect or the antibody-drug conjugate, pharmaceutically acceptable salt, stereoisomer or solvate described in the second aspect in preparation of a medicament for treating a disease associated with an abnormal cell activity, such as a cancer disease.

In a sixth aspect, the present disclosure provides a use of the compound, pharmaceutically acceptable salt, stereoisomer or solvate described in the first aspect, or the antibody-drug conjugate, pharmaceutically acceptable salt, stereoisomer or solvate described in the second aspect, or the pharmaceutical composition described in the fourth aspect in treatment of a disease associated with an abnormal cell activity, such as a cancer disease.

In a seventh aspect, the present disclosure provides a method for treating a disease associated with an abnormal cellular activity, such as a cancer disease, including: administering to an individual in need thereof an effective dose of the compound, pharmaceutically acceptable salt, stereoisomer or solvate described in the first aspect, or the antibody-drug conjugate, pharmaceutically acceptable salt, stereoisomer or solvate described in the second aspect, or the pharmaceutical composition described in the fourth aspect. The dosage regimen can be adjusted to provide the best desired response. For example, a single bolus can be administered, several divided doses can be administered over time, or the dose can be proportionally reduced or increased as indicated by the urgent need for the treatment situation. The dose value may vary with the type and severity of the illness condition to be alleviated and may include single or multiple doses. It should be further understood that for any particular individual the specific dosage regimen should be adjusted over time according to the individual's needs and the professional judgment of the person administering the composition or supervising the administration of the composition.

Various embodiments may involve use of the subject method and composition to treat a cancer, including, but not limited to, metastatic breast cancer, non-small cell lung cancer, Burkitt lymphoma, Hodgkin's lymphoma, acute myeloid leukemia , chronic myelogenous leukemia, acute lymphocytic leukemia, chronic lymphocytic leukemia, skin cancer, oral cancer, esophageal cancer, gastrointestinal cancer, lung cancer, lung cancer, gastric cancer, colon cancer, rectal cancer, triple negative breast cancer, ovarian cancer, prostate cancer, uterine cancer, endometrial cancer, cervical cancer, bladder cancer, pancreatic cancer, bone cancer, brain cancer, connective tissue cancer, thyroid cancer, liver cancer, gallbladder cancer, bladder (urothelial) cancer, kidney cancer, skin cancer, central nervous system cancer, and testicular cancer.

In some embodiments involving the treatment of cancers, the antibodies or immunoconjugates may be used in combination with surgery, radiation therapy, chemotherapy, immunotherapy with naked antibodies including inhibitory antibodies of checkpoint, radioimmunotherapy, immunomodulators, vaccines, etc. Most preferably, the antibodies or immunoconjugates are used in combination with PARP inhibitors, microtubule inhibitors, Bruton's kinase inhibitors and/or PI3K inhibitors. These combined therapies may allow lower doses of the therapeutic agents to be administered in the context of the combinations, thereby reducing some of the severe side effects and potentially reducing the duration of treatment required. The respective full doses may also be administered when there is no or minimal overlapping toxicity.

Although the antibody or immunoconjugate may be administered as a periodic bolus injection, in alternative embodiments, the antibody or immunoconjugate may be administered by continuous infusion. To increase the Cmax of the antibody or immunoconjugate in the blood and prolong the PK of the antibody or immunoconjugate in the blood, continuous infusion can be implemented, for example, through an indwelling catheter. Such devices are known in the art, such as HICKMAN^{®}, BROVIAC^{®}, or Port-A-Cath^{®} catheter (see, e.g., Skolnik et al., Ther Drug Monit 32:741-48, 2010), and any such known indwelling catheter may be used. A variety of continuous infusion pumps are also known in the art, and any such known infusion pump may be used.

In particularly preferred embodiments, the antibody or immunoconjugate and the dosing schedule may be effective in patients who are resistant to standard therapies. For example, a hRS7-eribulin immunoconjugate can be administered to a patient who has not responded to prior therapy with eribulin. The ability of an immunoconjugates to specifically target tumor tissue may overcome tumor resistance due to improved targeting and enhanced delivery of therapeutic agents. A particularly preferred subject may be a patient with Trop-2 positive breast cancer, ovarian cancer, cervical cancer, endometrial cancer, lung cancer, prostate cancer, colon cancer, rectal cancer, gastric cancer, esophageal cancer, bladder (urothelial) cancer, kidney cancer, pancreatic cancer, brain cancer, thyroid cancer, epithelial cancer, or head and neck cancer. Preferably, the cancer is a metastatic cancer. More preferably, the patient has previously failed treatment with at least one standard anti-cancer therapy.

In an eighth aspect, the present disclosure provides a pharmaceutical preparation, comprising the compound, pharmaceutically acceptable salt, stereoisomer or solvate described in the first aspect, or the antibody-drug conjugate, pharmaceutically acceptable salt, stereoisomer or solvate described in the second aspect, or the pharmaceutical composition described in the fourth aspect.

In a ninth aspect, the present disclosure provides a use of the compound, pharmaceutically acceptable salt, stereoisomer or solvate described in the first aspect, or the antibody-drug conjugate, pharmaceutically acceptable salt, stereoisomer or solvate described in the second aspect, or the pharmaceutical composition described in the fourth aspect in preparation of a pharmaceutical preparation.

In a tenth aspect, the present disclosure provides a kit, comprising the compound, pharmaceutically acceptable salt, stereoisomer or solvate described in the first aspect, or the antibody-drug conjugate, pharmaceutically acceptable salt, stereoisomer or solvate described in the second aspect, or the pharmaceutical composition described in the fourth aspect, or the pharmaceutical preparation described in the eighth aspect.

The compounds, conjugates, pharmaceutically acceptable salts, stereoisomers, or solvates, pharmaceutical preparations or kits provided by the present disclosure can be used for inhibiting the growth, proliferation or migration of a cancer cell. The compounds, conjugates, pharmaceutically acceptable salts, stereoisomers, or solvates, pharmaceutical preparations or kits are for in vivo or in vitro administration, for example, administered into the body of an individual, or to cells in vitro (e.g., a cell line or cells from an individual, such as cancer cells).

Therefore, in the eleventh aspect, the present disclosure provides a method for inhibiting the growth, proliferation or migration of a cancer cell, including: administering to the cancer cell an effective dose of the compound, pharmaceutically acceptable salt, stereoisomer or solvate described in the first aspect, or the antibody-drug conjugate, pharmaceutically acceptable salt, stereoisomer or solvate described in the second aspect, or the pharmaceutical composition described in the fourth aspect, or the pharmaceutical preparation described in the eighth aspect.

Unless otherwise defined, the meanings of all technical and scientific terms used herein are intended to be the same as those commonly understood by those skilled in the art. The reference to the technology used herein is intended to refer to the technology generally understood in the art, including those technical changes or equivalent technology substitutions that are obvious to those skilled in the art. Although it is believed that the following terms are well understood by those skilled in the art, the following definitions are still set forth to better explain the disclosure.

The "connector", "connector structure" or "linker" or "linking unit" mentioned in the present invention refers to a chemical structural fragment or bond that is linked to an antibody at one end and to a drug (drug compound) at the other end, and may also be linked to other linker(s) and then linked to the pharmaceutical compound. The connector structure of the present invention may be synthesized by methods known in the art, or may be synthesized using the method described herein.

The "antibody-drug conjugate" of the present invention, namely ADC, refers to a targeting moiety linked to a biologically active drug via a stable linking unit.

The term "pharmaceutically acceptable salt", as used herein, refers to a relatively nontoxic acid addition salt or base addition salt of the conjugate of the present invention. The acid addition salt refers to a salt formed by the conjugate of the present invention and a suitable inorganic or organic acid. These salts may be prepared by reacting the conjugate of the present invention with a suitable organic or inorganic acid in a suitable solvent. Representative acid addition salts include hydrobromide, hydrochloride, sulfate, hydrogen sulfate, sulfite, acetate, oxalate, valerate, oleate, palmitate, stearate, lunar silicate, borate, benzoate, lactate, nitrate, phosphate, hydrogen phosphate, carbonate, bicarbonate, toluate, citrate, maleate, fumarate, succinate, malate, ascorbate, tannate, pamoate, alginate, naphthalene sulfonate, tartrate, benzoate, methanesulfonate, p-toluenesulfonate, gluconate, lactobionate and lauryl sulfonate. The base addition salt is a salt formed by the conjugate of the present invention and a suitable inorganic or organic base. These salts may be prepared by reacting the conjugate of the present invention with a suitable inorganic or organic base in a suitable solvent. Representative base addition salts include, for example, salts formed by reaction with alkali metal, alkaline earth metal, and quaternary ammonium cations, such as sodium salts, lithium salts, potassium salts, calcium salts, magnesium salts, tetramethyl quaternary ammonium salts, tetraethyl quaternary ammonium salts; amine salts, including salts formed by reaction with ammonia (NH₃), primary amine, secondary amine or tertiary amine, such as methylamine salts, dimethylamine salts, trimethylamine salts, triethylamine salts, ethylamine salts.

The conjugate of the present invention may exist in specific geometric or stereoisomer forms. In the conjugate of the present invention, a chiral center may exist in the drug, in the connector structure, or in the antibody and its derivative. In the present invention, all such compounds, including cis- and trans-isomers, (-)- and (+)-enantiomers, (R)- and (S)-enantiomers, diastereomers, (D)-isomers, (L)-isomers, and their racemic mixtures and other mixtures, such as enantiomerically or diastereomerically enriched mixtures, are included within the scope of the present invention.

The optically active (R)- and (S)-isomers as well as the D and L isomers may be prepared by chiral synthesis or chiral reagents or other conventional techniques. If it is desired to obtain an enantiomer of the conjugate of the invention, the enantiomer may be prepared by asymmetric synthesis or derivatization with chiral reagents, in which the resulting diastereomer mixture is separated and the auxiliary group is cleaved to provide a pure product of the desired enantiomer. Alternatively, when the molecule contains a basic functional group (such as an amino group) or an acidic functional group (such as a carboxyl group), a salt of a diastereoisomer is formed by reaction with an appropriate optically active acid or base, and then the diastereoisomer is resolved by conventional methods known in the art, and then a pure enantiomer is recovered and obtained. Furthermore, the separation of enantiomers and diastereomers is usually accomplished through use of chromatography, and the chromatography uses chiral stationary phases and is optionally combined with a chemical derivatization method (for example, carbamates are formed from amines).

In the present invention, solvates (such as hydrates) of the compounds, conjugates, pharmaceutically acceptable salts, and stereoisomersof the present invention are also within the scope of the present invention. As suitable solvates, specifically, solvates formed by reaction between the conjugate of the present invention and acetone, 2-butanol, 2-propanol, ethanol, ethyl acetate, tetrahydrofuran, diethyl ether, and the like may be recited. Hydrates or ethanolates may also be recited.

As used herein, "treating" an individual suffering from a disease or disease condition means that the symptoms of the individual are partially or completely relieved or remain unchanged after treatment. Therefore, the treating includes preventing, treating and/or curing. The preventing refers to the prevention of underlying diseases and/or prevention of symptoms from worsening or disease progression. The treating also includes any pharmaceutical use of the ADC provided as well as the pharmaceutical composition and pharmaceutical preparation provided herein.

The term "therapeutic effect", as used herein, means the effect resulting from treatment of an individual, which is a change, usually amendment or amelioration of symptoms of a disease or disease condition, or cure of a disease or disease condition.

The term "individual", as used herein, includes human or non-human animals. Exemplary human individuals include human individuals (referred to as patients) suffering from diseases such as those described herein and normal individuals. The term "non-human animal", as used herein, includes all vertebrates, such as non-mammals (such as birds, amphibians, and reptiles) and mammals, such as non-human primates, livestock and/or domesticated animals (such as sheep, dogs, cats, cows, and pigs).

It should be understood that the disclosed compositions and methods are not limited to the specific compositions and methods described and/or illustrated herein, and that the terms used herein are for the purpose of describing specific embodiments by way of example only and are not intended to limit the claimed compositions and methods.

Throughout this document, compositions and methods of using the compositions are described. Where the present disclosure describes or claims features or embodiments in connection with a composition, such features or embodiments apply equally to methods of using the composition. Likewise, where the present disclosure describes or claims features or embodiments in connection with methods of using a composition, such features or embodiments apply equally to the composition.

When a range of values is stated, it includes embodiments using any specific value within the stated range. Furthermore, references to values recited in a range include every value within that range. All ranges include their endpoints and can be combined. When values are expressed as approximations, by use of the preceding word "about," it is understood that the particular value forms an alternative embodiment. Unless the context clearly dictates otherwise, references to a specific numerical value include at least the stated specific value. The use of "or" will mean "and/or" unless the specific context in which it is used dictates otherwise. All references cited herein are incorporated by reference for any purpose. In the event of a conflict between a reference and this specification, this specification will control.

It is understood that, for clarity, certain features of the disclosed compositions and methods that are described herein in the context of separate embodiments may also be provided in combination in a single embodiment. Conversely, for simplicity, various features of the disclosed compositions and methods that are described in the context of a single embodiment may also be provided separately or in any subcombination.

References mentioned herein are incorporated by reference in their entirety.

The embodiments of the invention will be described in detail below in conjunction with the accompanying drawings and embodiments. However, those skilled in the art will understand that the following drawings and embodiments are only for illustrating the invention, not to limit the scope of the invention. Various objectives and advantageous aspects of the invention will become apparent to those skilled in the art according to the accompanying drawings and the following detailed description of the preferred embodiments.

### BRIEF DESCRIPTION OF THE DRAWINGS

Fig. 1 shows the SEC-HPLC results of conjugate ADC-4 synthesized in Example 2.
Fig. 2 shows statistical curves of tumor-inhibitory activity in Example 5.

### DETAILED DESCRIPTION OF EMBODIMENTS

The solution of the present invention will be explained below with reference to specific embodiments. It should be understood that these embodiments are only for illustrating the invention by example and not to limit the scope of the invention. Where specific techniques or conditions are not specified in the following embodiments, the techniques or conditions described in documents in the field or product instructions should be followed. The reagents or instruments used without specifying the manufacturers are all conventional products that can be purchased commercially.

In the following examples, ADC-137 was only used as a control. The linker-toxin small molecule moiety was purchased from Changzhou Chenhong Biotechnology Co., Ltd., CAS#: 1279680-68-0. The antibody conjugating method was the same as that of ADC-4 below. The structure of ADC-137 is represented by:

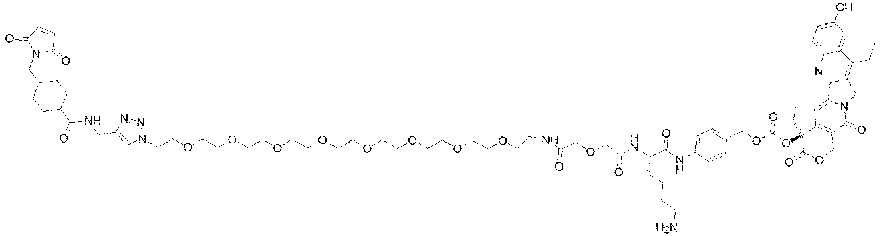

### Example 1: Preparation and testing of hRS7 antibodies

### 1. Gene synthesis, transfection and antibody preparation

The hRS7 antibodies were produced in CHO cells. Expression vectors containing the hRS7 antibody gene were constructed using conventional molecular biology methods. The amino acid sequences of the light chain and heavy chain in the hRS7 antibody are as shown in SEQ ID NO: 1 and SEQ ID NO: 2 respectively, and their corresponding nucleotide sequences are as shown in SEQ ID NO: 3 and SEQ ID NO: 4, respectively. The above two sequences were inserted into the same expression vector, a large amount of transfection plasmids were extracted and prepared, and transfected into CHO-K1 cells (ATCCCCL-61). The specific transfection and antibody preparation processes were as follows:
(1) Cell culture: CHO-K1 cells were grown in suspension in ActiPro (GE HyClone) medium and cultured at 37 °C, 7% CO₂, 140 rpm, and a relative humidity of 90%.
(2) Transfection: After entering the logarithmic growth phase, the cells were centrifuged, resuspended in fresh ActiPro medium and counted and the cell density was adjusted to 1.2×10⁷ cells/mL. 500 µl of cell suspension was transfered into an electroporation cup, and then 40 µg of constructed plasmids were added. The cells and plasmids were well mixed, and then the plasmids were introduced by electroporation (Bio-rad electroporator).
(3) Subcloning: The cells after electroporation were resuspended in ActiPro medium at 37 °C, and then dispensed into 96-well plates with 100 µl per well. Cell supernatants were assayed to determine antibody expression levels. The clones with high expression levels were transferred from the 96-well plates to 24-well plates for culture, and then transferred to 6-well plates for culture. The antibody production and yield of the cells were measured, and four clones with the highest expression levels were selected for subcloning, and then transferred to shake flasks and further cultured in an incubator.

### 2. Purification of antibodies

The highly expressed cell fluid cultured in shake flasks was harvested and subjected to affinity purification with protein A (GE, Mab Select SuRe) and ion exchange purification (GE, Capto S). SDS-PAGE and SEC-HPLC were implemented to analyze the molecular weight and purity of the purified antibodies. The SDS-PAGE measurement results showed that the molecular weight of the prepared hRS7 was in line with expectations, and the antibody purity measured by SEC-HPLC was 98.8%.

### Example 2: Preparation of ADC-4

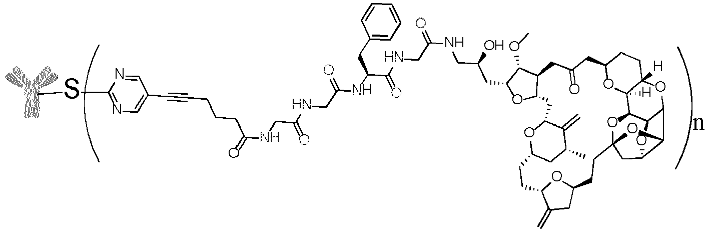

### 1. Preparation of intermediate D

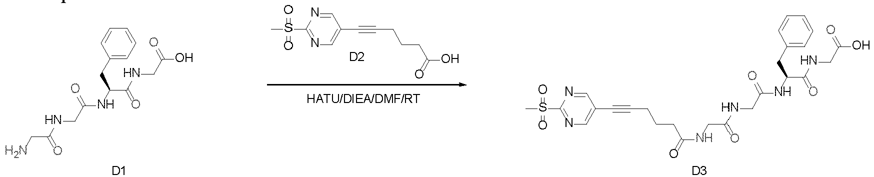

1.1 To a solution of 6-(2-(methylsulfonyl)pyrimidine)-5-hexynoic acid (39.88 mg) D2 in DMF (4 mL) were added HATU (56.52 mg) and DIEA (38.43 mg). The reaction solution was stirred at room temperature for 2 h, compound D1 (50 mg) was then added, and the reaction solution was stirred at room temperature for 3 h. HPLC showed the formation of a new peak. The reaction solution was purified by Pre-HPLC and then lyophilized to give the product (about 21 mg) as a white solid D3, with a yield of about 24.08%. LCMS:[M+1]+=587.4.

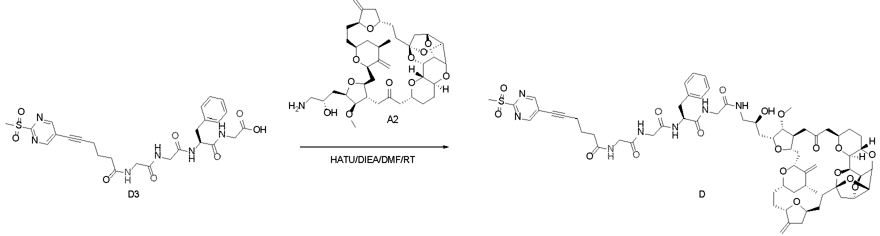

1.2 To a solution of compound D3 (21 mg) in DMF (4 mL) were added compound HATU (20.42 mg) and DIEA (9.25 mg). The reaction solution was stirred at room temperature for half an hour, then compound eribulin A2 (26.13 mg) was added, and the reaction solution was stirred at room temperature for 3 h. HPLC showed the formation of a new peak, and the reaction solution was purified by Pre-HPLC and then lyophilized to give the product D (about 11 mg) as a white solid, with a yield of about 23.66%. LCMS:[M+1]+=1298.9;

### 2. Synthesis of crude product ADC-4

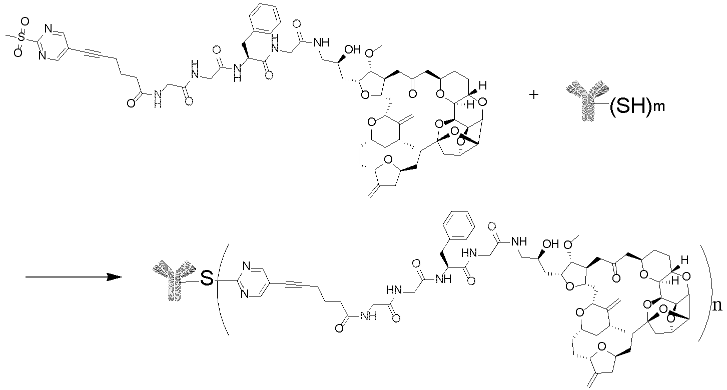

5 mM of solution of the antibody in 10 mg/mL pH 7.4 PBS/EDTA was placed in an ice water bath. When the temperature of the protein solution reached 4 °C, an equal volume of a solution of 3.5 times equivalent of TCEP was added with stirring and mixing. The solution was set still in a 25 °C water bath and allowed to react for 1 h. When the reaction solution in the 25°C water bath was cooled to 25 °C, a solution of 7 times equivalent compound D in 40% DMSO was added to the antibody solution with stirring and mixing. The reaction was allowed by shaking on a shaker at 25 °C for 90 min. Finally, 8 times equivalent of cysteine was added to the reaction solution with stirring and mixing. The solution was set still in a 25 °C water bath and allowed to react for 10 min to give the conjugate product ADC-4.

### 3. Testing of crude conjugate product ADC-4

The crude conjugate productwas test by SEC,
SEC was performed under the following conditions:
Column model: TSKgel G4000SWxl 7.8mmI.D.*30cm, 8µm
Tester wavelength: 280 nm/220 nm
Column temperature: 30 °C
Flow rate: 1 mL/min
Elution mode: Isocratic elution
Injection volume: 10 µL
Operation time: 55 min

### 4. Purification of conjugation reaction product:

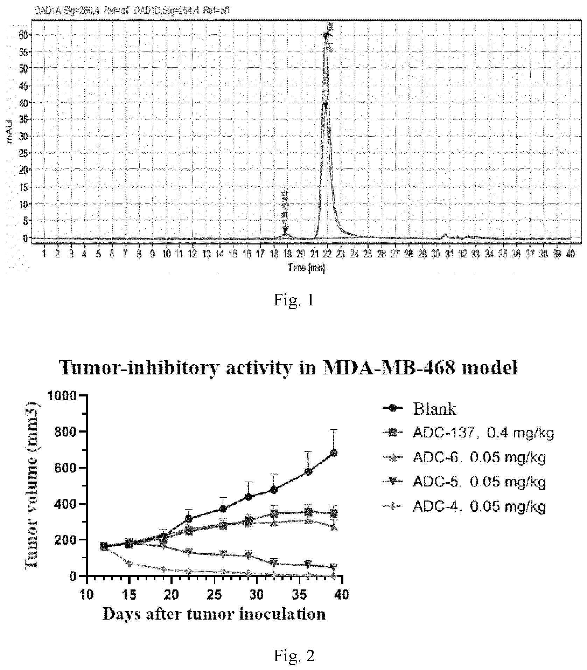

The crude product was purified by PD-10 desalting column (packing: sephadex G 25) to give a conjugate. The conjugate was then transferred to a solution of trehalose 5% in MES 25 Mm with pH 5.5. It was found by SEC that small molecules had been completely removed. The SEC-HPLC results of purified ADC-4 are shown in Figure 1.

### 5. Determination of DAR

To ADC-4 and the monoclonal antibody solution was added an equal volume of 50 mM dithiothreitol (DTT) solution, the solution was vortex mixed and placed in a 37 °C water bath for 30 min. The reduced ADC and antibody were analyzed by RP-HPLC for conjugated and unconjugated light chain and heavy chain of the antibody. The DAR was determined to be 4.2 by analyzing its composition.

### Example 3 Preparation of ADC-5

### 1 Preparation of intermediate E

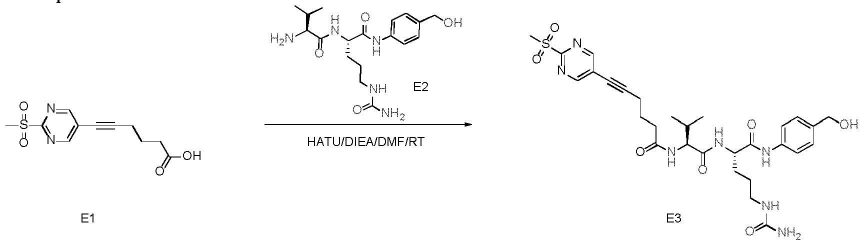

1. To a solution of E1 (141.4 mg) in DMF (8 mL) were added compounds HATU (300.6 mg) and DIEA (136.24 mg). The reaction solution was stirred at room temperature for half an hour. VC-PAB-OH E2 (200 mg) was then added, and the reaction solution was stirred at room temperature for 3 h. HPLC showed the completion of the reaction and the formation of a new peak. The reaction solution was purified by Pre-HPLC and then lyophilized to give the product E3 (about 155 mg) as a white solid, with a yield of about 46.7%. LCMS:[M+1]+=630.4; 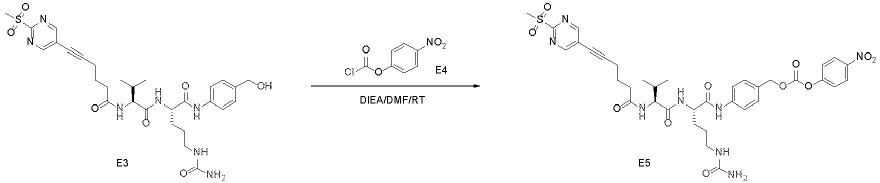
2. To a solution of E3 (155 mg) in DMF (6 mL) were added the compound p-nitrophenyl chloroformate E4 (74.42 mg) and DIEA (63.62 mg), and the reaction solution was stirred at room temperature for 3 h. HPLC showed the completion of the reaction and the formation of a new peak. The reaction solution was purified by Pre-HPLC and lyophilized to give the product E5 as a white solid (about 80 mg), with a yield of about 40.89%. LCMS:[M+1]+=795.4; 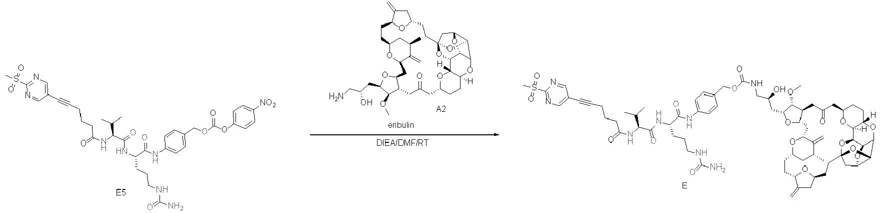
3. To a solution of E5 (25 mg) in DMF (3 mL) were added the compounds eribulin A2 (23 mg) and DIEA (8.13 mg), and the reaction solution was stirred at room temperature for 3 h. HPLC showed the completion of the reaction and the formation of a new peak. LCMS showed the formation of the target product. The reaction was purified by Pre-HPLC and then lyophilized to give the product E (about 11 mg) as a white solid, with a yield of about 25.24%. LCMS: [M+1]+=1385.9;
4. The steps of coupling E and the hRS7 antibody prepared in Example 1, purification, and testing were the same as steps 2-5 in Example 3. The DAR of the obtained ADC-5 was 4.2. That is, n is 4.2.

### Example 4 Preparation of ADC-6

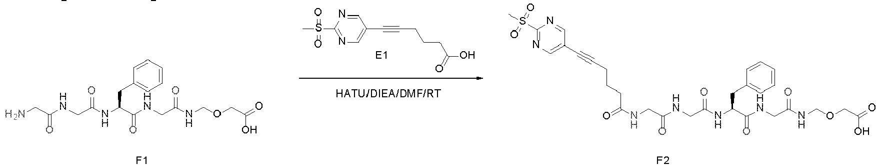

1. To a solution of 6-(2-(methylsulfonyl)pyrimidine)-5-hexynoic acid E1 (31.68 mg) in DMF (4 mL) were added compound HATU (44.9 mg) and DIEA (30.52 mg). The reaction solution was stirred at room temperature for 2 h, compound F1 (50 mg) was then added, and the reaction solution was stirred at room temperature for 3 h. HPLC showed the formation of a new peak, and the reaction solution was purified by Pre-HPLC and then lyophilized to give the product F2 (about 27 mg) as a white solid, with a yield of about 33.94%. LCMS:[M+1]+=674.4. 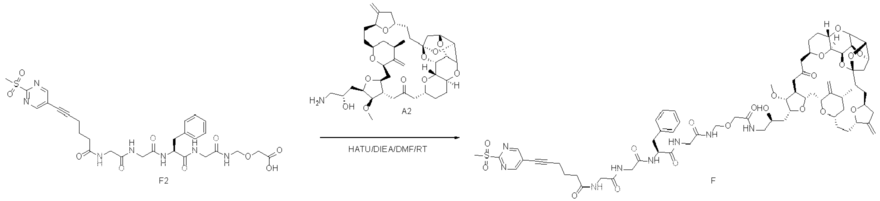
2. To a solution of compound F2 (27 mg) in DMF (4 mL) were added compound HATU (22.86 mg) and DIEA (10.36 mg). The reaction solution was stirred at room temperature for half an hour, compound eribulin A2 (29.25 mg) was then added, and the reaction solution was stirred at room temperature for 3 h. HPLC showed formation of a new peak. The reaction solution was purified by Pre-HPLC and then lyophilized to give the product F (about 13 mg) as a white solid, with a yield of about 23.41%. LCMS:[M+1]+=1385.9.
3. The steps of coupling F and the hRS7 antibody prepared in Example 1, purification, and testing were the same as steps 2-5 in Example 3. The DAR of the obtained ADC-6 was 4.3. That is, n is 4.3.

### Example 5 Tumor-inhibitory effect of ADC on breast cancer

### Experimental method

### 1.1 Cell culture

MDA-MB-468 (ATCC HTB-132) cells were incubated in monolayer in vitro in L-15 medium supplemented with 10% heat-inactivated fetal bovine serum at 37 °C in an incubator without CO₂ atmosphere. The liquid was changed 2-3 times a week. When the cells were in the exponential growth phase, digesting the cells by using 0.25% trypsin, and carrying out passage in a ratio of 1:2-1:4 were performed. After entering the logarithmic growth phase, the cells were harvested, counted and inoculated.

### 1.2 Tumor cell inoculation and passage of tumor mass

Five nude mice (P1) were inoculated at the right scapula with 4.5.0×10⁶ MDA-MB-468 tumor cells which were suspended in 0.1 ml of mixture of PBS and Matrigel (1:1). When tumor size reached 600-800 mm³, the tumor-bearing mice were killed under CO₂ anesthesia, the tumor mass was removed, the surrounding necrotic tissue was removed, the tumor mass was cut into small masses of 20-30 mm³, and then inoculated into a new batch of nude mice (P2).

### 1.3 Tumor inoculation and group administration

In this experiment, P6 tumor tissue was used to evaluate the anti-tumor activity of the test product. When the tumor in P5 had a size of 600-800 mm³, the tumor-bearing mice were killed under CO₂ anesthesia, the tumor mass was removed, the surrounding necrotic tissue was removed, the tumor mass with good state was cut into small masses of 20-30 mm³, and then inoculated into total 40 experimental mice at the right scapula. When the average tumor volume reached approximately 135 mm³ in 13 days after tumor inoculation, mice with tumors that were too small or too large were eliminated, and the remaining 20 mice were randomly grouped according to tumor volume and subjected to drug administration. The dosage regimen was shown in the table below.

| Gro up | Number of mice | Control sample/test sample | Dose (mg/k g) | Route of administration | Administration schedule |
|---|---|---|---|---|---|
| 1 | 5 | Blank (PBS) | -- | IV | Only one dose |
| 2 | 5 | ADC-4 | 0.05 | IV | Only one dose |
| 3 | 5 | ADC -5 | 0.05 | IV | Only one dose |
| 4 | 5 | ADC-6 | 0.05 | IV | Only one dose |
| 5 | 5 | ADC-137 | 0.4 | IV | Only one dose |

| | | | | | |
|---|---|---|---|---|---|
| Note: "only one dose" means administration with one dose on the day of grouping and no further administration; its dose was calculated according to the dose of eribulin in the ADC. | | | | | |

### 1.4 Experimental observations and data collection

After tumor cells were inoculated, in addition to observing tumor growth, the impact of drug treatment on animal behavior was also monitored, including mobility, food intake and drinking water, body weight change (body weight was measured twice a week), eyes hair and abnormalities of experimental animals. Clinical symptoms observed during the experiment were recorded in the raw data. The tumor volume was calculated as follows: tumor volume (mm³) = 1/2 × (a × b²) (a: long diameter; b: short diameter).

When the weight loss of a single animal exceeded 15% (BWL>15%), the corresponding single animal would be given drug withdrawal treatment. Once the weight loss returned to within 10%, drug administration would be resumed. When a single mouse losed >20% of its body weight, it would be euthanized in accordance with animal welfare.

### 1.5 Efficacy evaluation criteria

Percent of relative tumor proliferation, T/C%, is the percentage of relative tumor volume or tumor weight of the treatment group to the control group at a certain time point. It is calculated according to the formula below: T/C % = T_{RTV} / C_{RTV} × 100% (T_{RTV}: average RTV of the treated group; C_{RTV}: average RTV of the vehicle control group; RTV=Vₜ/V₀, V₀ is the tumor volume of the animal when grouped, Vₜ is the tumor volume of the animal after treatment); or T/C % = T_{TW} / C_{TW} × 100% (T_{TW}: average tumor weight of the treated group at the end of the experiment; C_{TW}: average tumor weight of the vehicle control group at the end of the experiment).

### 1.6 Experimental endpoint

One week after the last administration, tumors were removed from all mice, weighed, and photographed.

### 1.7 Statistical analysis

In this experiment, one-way ANOVA was used to compare the tumor mean values among the groups. Homogeneity of variance analysis showed significant differences in F values. After ANOVA analysis, Dunnet's T3 (heterogeneity of variance) was used for multiple comparisons. All data analyzes were performed using SPSS17.0. p<0.05 is considered to be a significant difference.

### Experimental results

Fig. 2 shows statistical curves of tumor-inhibitory activity. As shown in the figure, in this experiment, the antitumor activity of ADC-4 and ADC-5 is better than that of ADC-137.

The embodiments mentioned above are merely preferred embodiments of the invention and not intended to limit the invention. Any of modifications, equivalent substitutions, improvements, etc. made within the spirit and principle of the invention shall be covered in the protection scope of the invention.

## Claims

1. A compound of formula (I), a pharmaceutically acceptable salt thereof, a stereoisomer thereof, or a solvate of the compound, the pharmaceutically acceptable salt or the stereoisomer,
L¹-L²-L³-L⁴-D (I)
wherein L¹ is 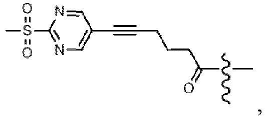 L² is a single bond or -NH-R¹-(CO)-,
or L¹-L² is 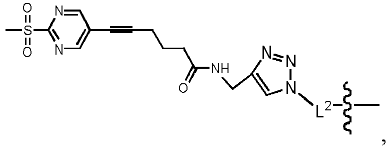 wherein L² is -R¹-(CO)-;
R¹ is selected from -(CH₂)ₐ-, -(CH₂CH₂O)_{b}-(CH₂)_{c}-, and -CH₂CH₂-(OCH₂CH₂)_{b}-NH-(CO)-CH₂-O-CH₂-;
L³ is a peptide residue, selected from the group consisting of glycine-glycine-phenylalanine-glycine (GGFG), glutamic acid-valine-citrulline (EVC), valine-citrulline (VC), aspartic acid-valine-citrulline (DVC), glutamic acid-glycine-glycine-phenylalanine-glycine (EGGFG), and aspartic acid-glycine-glycine-phenylalanine-glycine (DGGFG), the structural formulas are respectively: 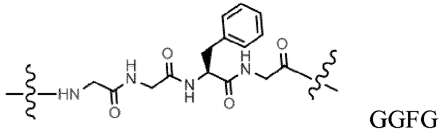 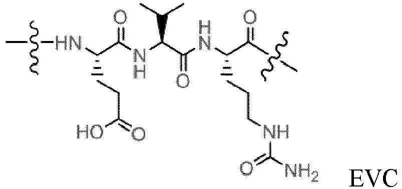 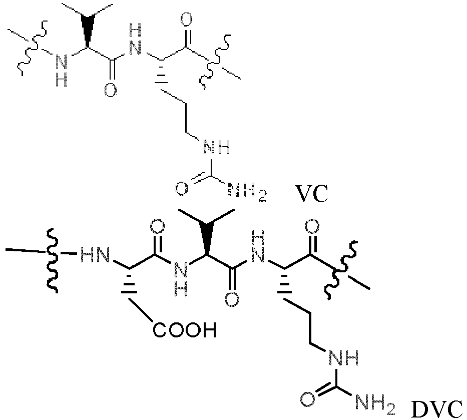 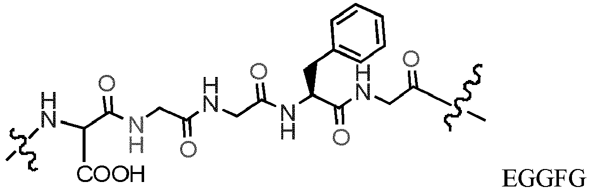 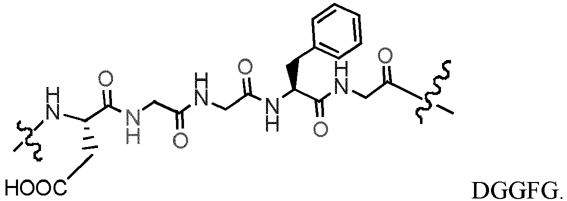
L⁴ is selected from the group consisting of a single bond, -NH-CH₂-, and 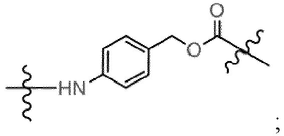
D is a drug linked to L⁴ via a chemical bond, and the drug is selected from the group consisting of eribulin or its derivative, camptothecin drugs, auristatins (such as monomethyl auristatin E (MMAE), and monomethyl auristatin F (MMAF));
a is selected from an integer between 1 and 6, such as, 1, 2, 3, 4, 5, and 6;
b is independently selected from an integer between 1 and 20, such as 1, 2, 3, 4, 5, 6, 7, 8, 9, 10, 11, 12, 13, 14, 15, 16, 17, 18, 19, and 20, for example, selected from an integer between 1 and 8;
c is 1 or 2.

2. The compound, pharmaceutically acceptable salt thereof, stereoisomer thereof, or a solvate of the compound, the pharmaceutically acceptable salt or the stereoisomer according to claim 1, wherein L³ is selected from GGFG, EVC, and VC.

3. The compound, pharmaceutically acceptable salt thereof, stereoisomer thereof, or a solvate of the compound, the pharmaceutically acceptable salt or the stereoisomer according to claim 1 or 2, wherein
L¹ is 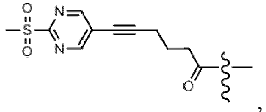 L² is -NH-R¹-(CO)-, R¹ is -(CH₂)ₐ-, and a is 2, 3, 4, 5 or 6, or
L¹ is 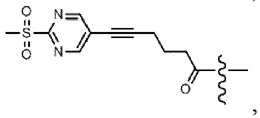 L² is -NH-R¹-(CO)-, R¹ is -(CH₂CH₂O)_{b}-(CH₂)_{c}-, b is 2, 3, 4, 5, 6, 7 or 8, and c is 1 or 2; or
L¹ is 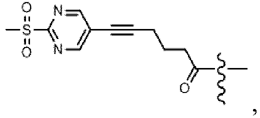 and L² is a single bond; or
L¹ is 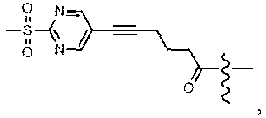 L² is -NH-R¹-(CO)-, R¹ is -CH₂CH₂-(OCH₂CH₂)_{b}-NH-(CO)-CH₂-O-CH₂-, and b is 2, 3, 4, 5, 6, 7 or 8; or
L¹-L² is 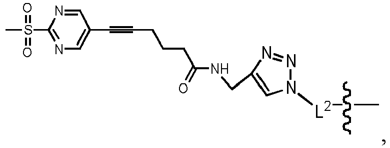 wherein L² is -R¹-(CO)-, and R¹ is -CH₂CH₂-(OCH₂CH₂)_{b}-NH-(CO)-CH₂-O-CH₂-, b is 2, 3, 4, 5, 6, 7 or 8; or
L¹ is 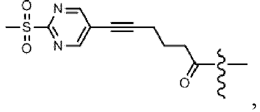 L² is -NH-R¹-(CO)-, R¹ is -(CH₂)ₐ-, a is 2, 3, 4, 5 or 6, and L³ is GGFG; or
L¹ is 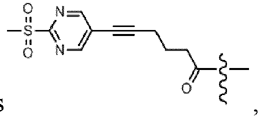 L² is -NH-R¹-(CO)-, R¹ is -(CH₂CH₂O)_{b}-(CH₂)_{c}-, b is 2, 3, 4, 5, 6, 7 or 8, c is 1 or 2, and L³ is GGFG or EVC; or
L¹ is 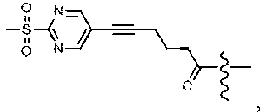 L² is a single bond, and L³ is GGFG; or
L¹ is 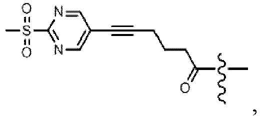 L² is -NH-R¹-(CO)-, R¹ is -CH₂CH₂-(OCH₂CH₂)_{b}-NH-(CO)-CH₂-O-CH₂-, b is 2, 3, 4, 5, 6, 7 or 8, and L³ is EVC; or
L¹-L² is 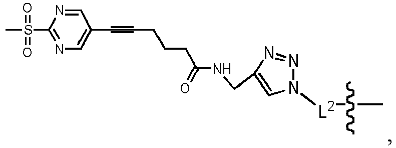 wherein L² is -R¹-(CO)-, R¹ is -CH₂CH₂-(OCH₂CH₂)_{b}-NH-(CO)-CH₂-O-CH₂-, b is 2, 3, 4, 5, 6, 7 or 8, and L³ is EVC; or
L¹ is 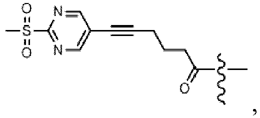 L² is a single bond, and L³ is VC or GGFG.

4. The compound, pharmaceutically acceptable salt thereof, stereoisomer thereof, or a solvate of the compound, the pharmaceutically acceptable salt or the stereoisomer according to any one of claims 1-3, wherein
L² is selected from the group consisting of a single bond,

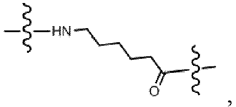

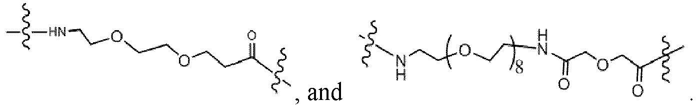

5. The compound, pharmaceutically acceptable salt thereof, stereoisomer thereof, or a solvate of the compound, the pharmaceutically acceptable salt or the stereoisomer according to claim 1 or 2, wherein
L¹-L² is

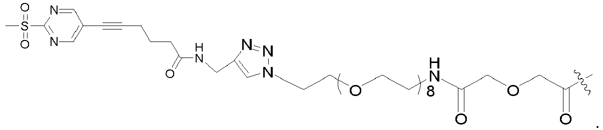

6. The compound, pharmaceutically acceptable salt thereof, stereoisomer thereof, or a solvate of the compound, the pharmaceutically acceptable salt or the stereoisomer according to any one of claims 1-5, wherein
L³ is GGFG, and L⁴ is a single bond; or
L³ is GGFG, and L⁴ is -NH-CH₂-; or
L³ is EVC, and L⁴ is 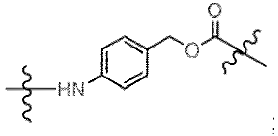 or
L³ is GGFG, and L⁴ is

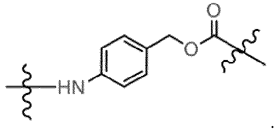

7. The compound, pharmaceutically acceptable salt thereof, stereoisomer thereof, or a solvate of the compound, the pharmaceutically acceptable salt or the stereoisomer according to any one of claims 1-6, wherein D is eribulin or its derivative;
preferably, D is selected from the group consisting of 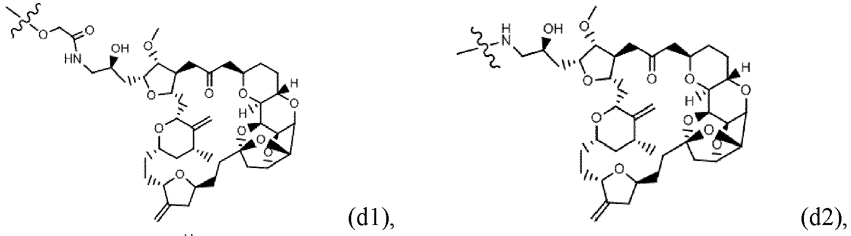 and

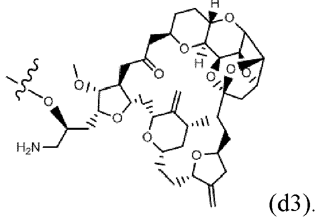

8. The compound, pharmaceutically acceptable salt thereof, stereoisomer thereof, or a solvate of the compound, the pharmaceutically acceptable salt or the stereoisomer according to any one of claims 1-5 and claim 7, wherein
L⁴ is -NH-CH₂-, and D is d1; or
L⁴ is -NH-CH₂-, and D is d3; or
L⁴ is a single bond, and D is d2; or
L⁴ is 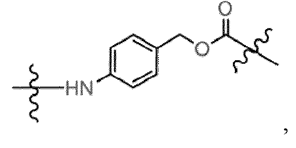 , and D is d2.

9. The compound, a pharmaceutically acceptable salt thereof, a stereoisomer thereof, or a solvate of the compound, the pharmaceutically acceptable salt or the stereoisomer according to any one of claims 1-8, wherein the compound is selected from:

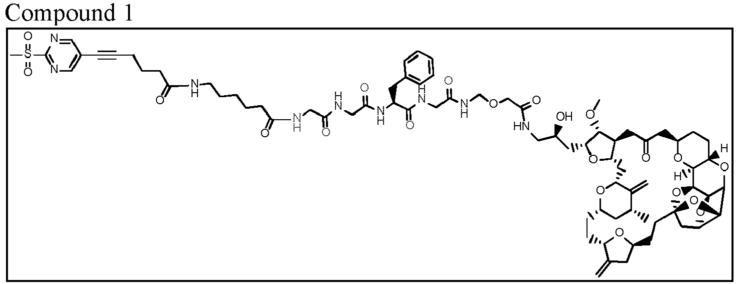

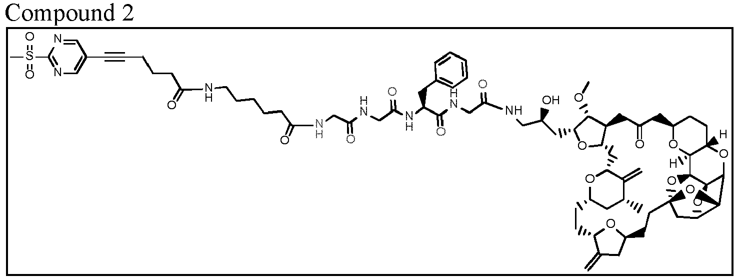

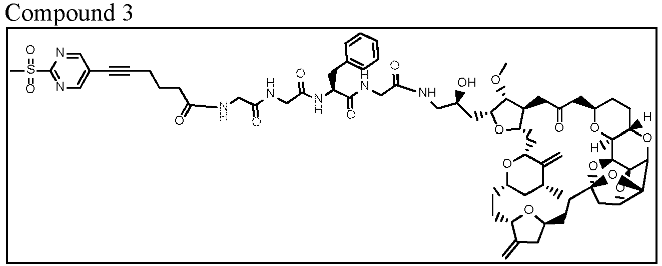

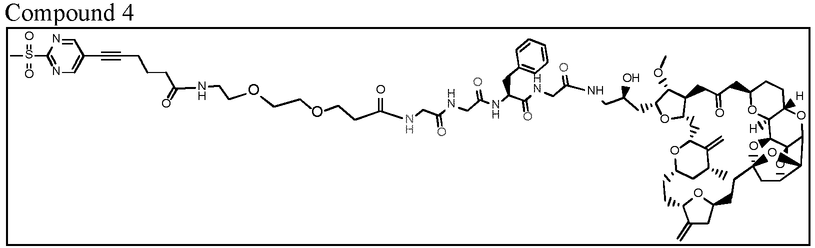

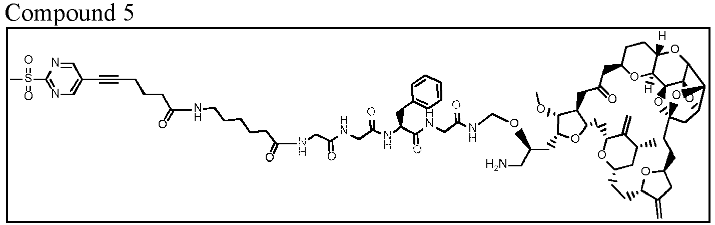

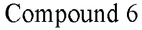

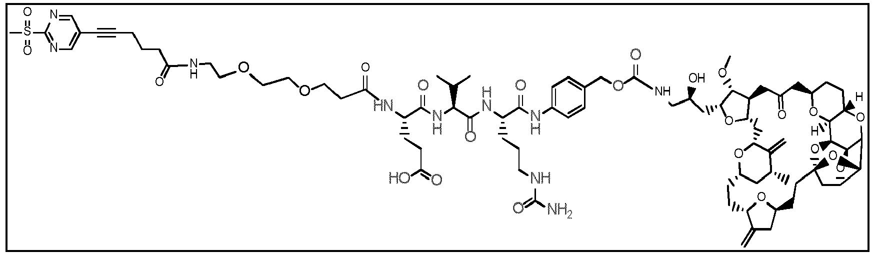

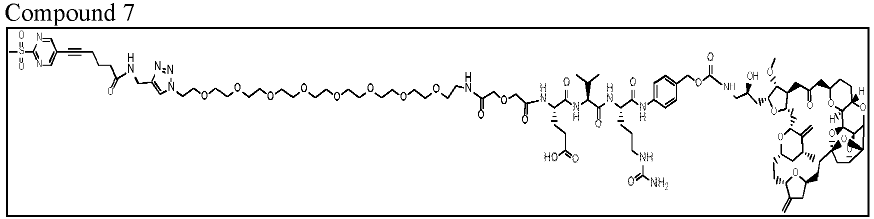

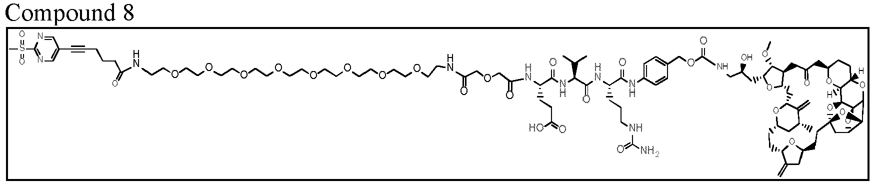

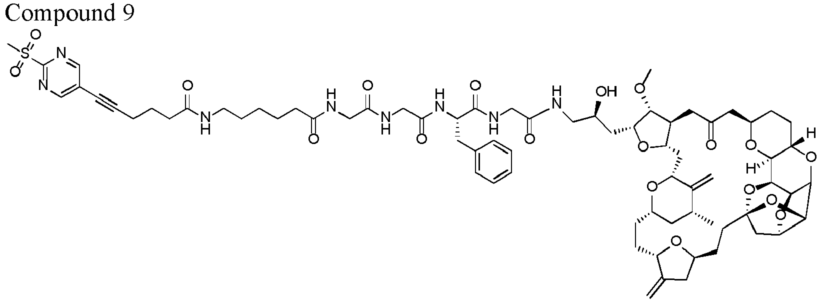

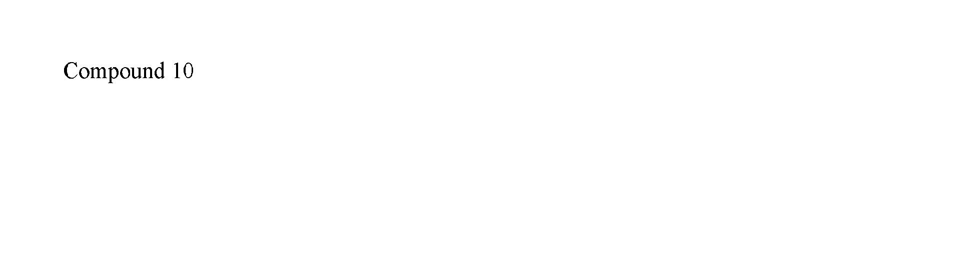

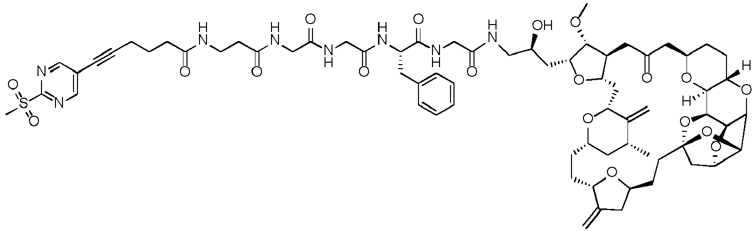

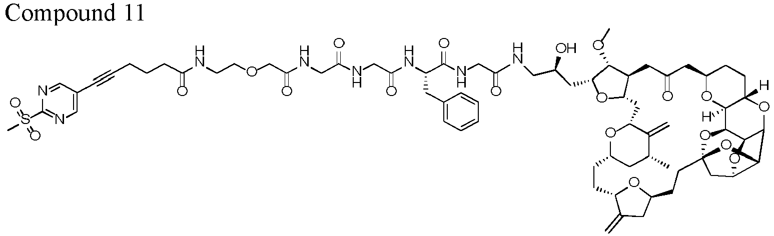

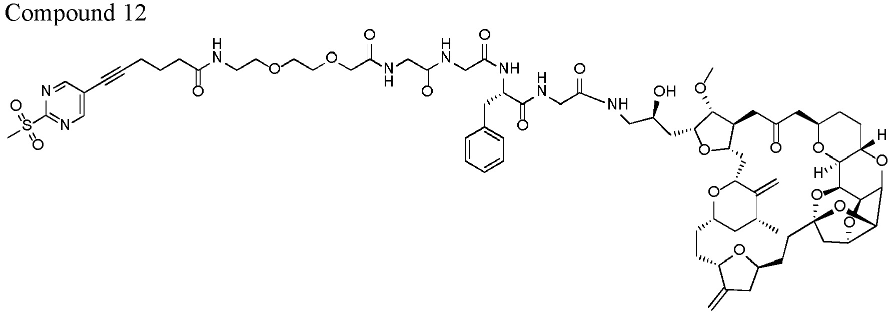

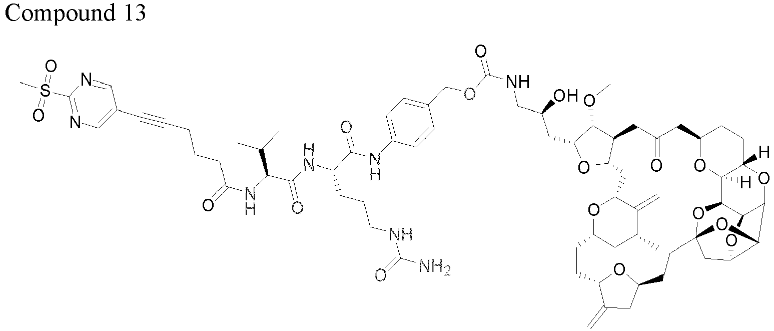

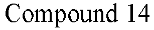

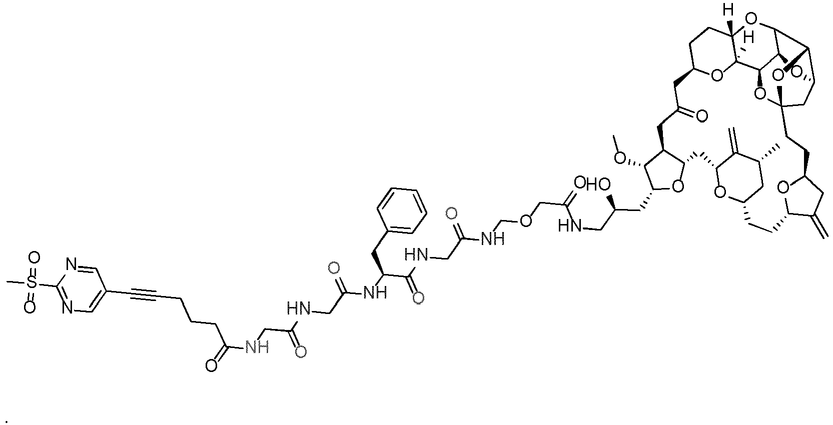

10. An antibody-drug conjugate, a pharmaceutically acceptable salt thereof, a stereoisomer thereof, or a solvate of the conjugate, the pharmaceutically acceptable salt or the stereoisomer, wherein the antibody-drug conjugate being formed by linking a targeting moiety to the compound of formula (I), a pharmaceutically acceptable salt thereof, a stereoisomer thereof or a solvate of the conjugate, the pharmaceutically acceptable salt or the stereoisomer via a thioether linkage;
preferably, the antibody-drug conjugate has a structure of formula (II)
Ab-(S-L¹-L²-L³-L⁴-D)ₚ (II)
wherein Ab is a targeting moiety selected from an antibody, an antibody fragment, or an antibody-based molecule or compound;
-S- is a thioether linkage,
L¹, L², L³, L⁴, and D are defined as any one of claims 1-8,
p is an integer between 1 and 20, such as 1, 2, 3, 4, 5, 6, 7, 8, 9, 10, 11, 12, 13, 14, 15, 16, 17, 18, 19, and 20, for example, selected from an integer between 1 and 8;
preferably, the Ab is an antibody, an antibody fragment, a bispecific or other multivalent antibody, or other antibody-based molecule or compound.

11. A preparation method of the compound, a pharmaceutically acceptable salt thereof, a stereoisomer thereof, or a solvate of the compound, the pharmaceutically acceptable salt or the stereoisomer according to any one of claims 1-9:
if L² is a single bond and L⁴ is 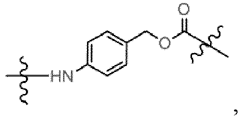 the method comprises the following steps: 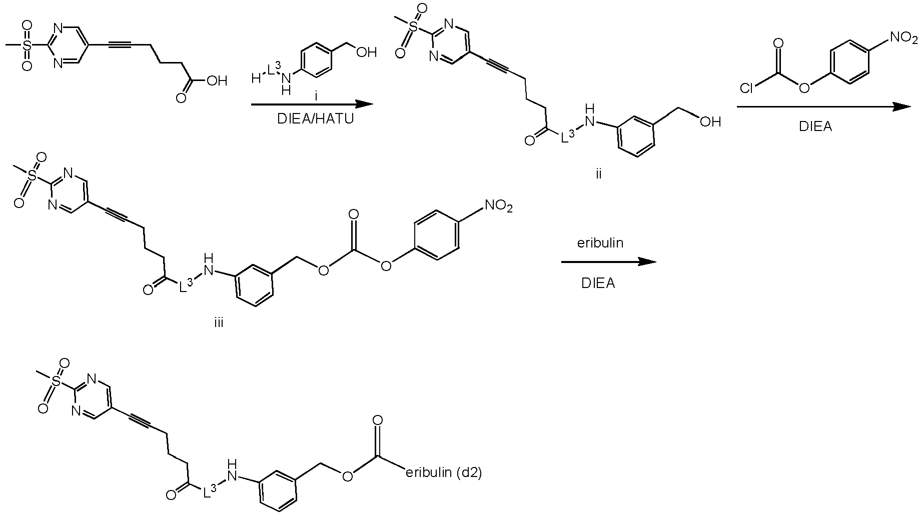
(1) allowing 6-(2-(methylsulfonyl)pyrimidine)-5-hexynoic acid and Compound i to have a condensation reaction in the presence of DIEA and HATU to generate Compound ii;
(2) allowing Compound ii and p-nitrophenyl chloroformate to have a condensation reaction in the presence of DIEA to generate Compound iii; and
(3) allowing Compound iii and eribulin to have a condensation reaction in the presence of DIEA to generate the compound of formula (I) (D is d2);
if L² is a single bond and L⁴ is -NH-CH₂-, the method comprises the following steps: 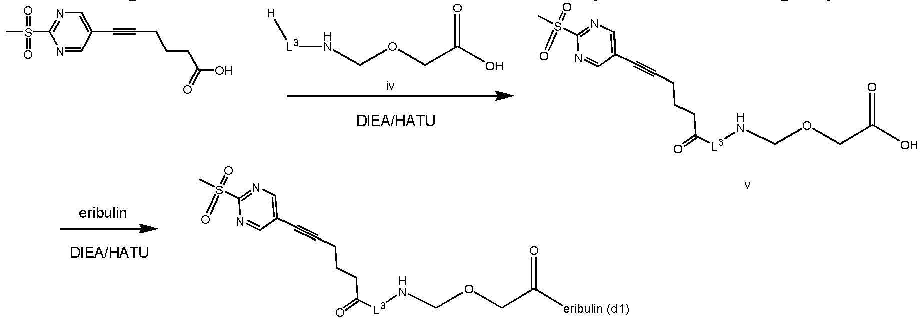
(1) allowing 6-(2-(methylsulfonyl)pyrimidine)-5-hexynoic acid and Compound IV to have a condensation reaction in the presence of DIEA and HATU to generate Compound V; and
(2) allowing Compound V and eribulin to have a condensation reaction in the presence of DIEA and HATU to generate the compound of formula (I) (D is d2);
if L² is a single bond and L⁴ is a single bond, the method comprises the following steps: 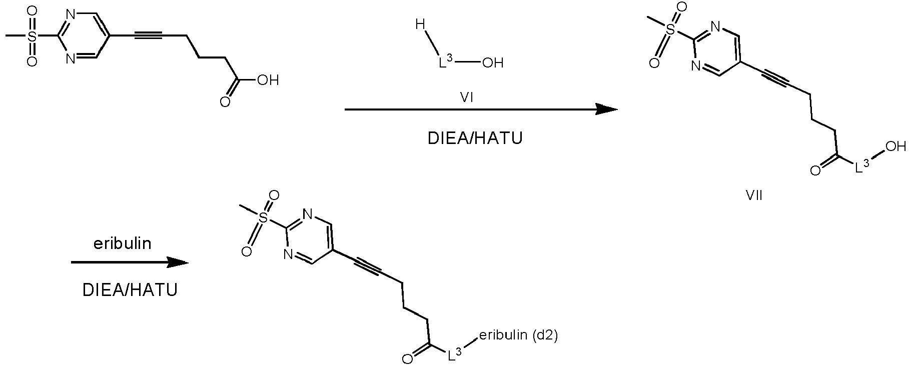
(1) allowing 6-(2-(methylsulfonyl) pyrimidine)-5-hexynoic acid and Compound VI to have a condensation reaction in the presence of DIEA and HATU to generate Compound VII; and
(2) allowing Compound VII and eribulin to have a condensation reaction in the presence of DIEA and HATU to generate the compound of formula (I) (D is d2).

12. A preparation method of the antibody-drug conjugate, a pharmaceutically acceptable salt thereof, a stereoisomer thereof, or a solvate of the conjugate, the pharmaceutically acceptable salt or the stereoisomer according to claim 10, comprising allowing the compound of formula (I), a pharmaceutically acceptable salt thereof, a stereoisomer thereof, or a solvate of the compound, the pharmaceutically acceptable salt or the stereoisomer to react with a targeting moiety (e.g., an antibody) such that a thioether linkage is formed by the disulfide bond portion of the hinge region in the targeting moiety.

13. A pharmaceutical composition, comprising the compound, a pharmaceutically acceptable salt thereof, a stereoisomer thereof, or a solvate of the compound, the pharmaceutically acceptable salt or the stereoisomer according to any one of claims 1-9, or the antibody-drug conjugate, a pharmaceutically acceptable salt thereof, a stereoisomer thereof, or a solvate of the conjugate, the pharmaceutically acceptable salt or the stereoisomer according to claim 10, and one or more pharmaceutical excipients, such as carriers and/or excipients.

14. Use of the compound, a pharmaceutically acceptable salt thereof, a stereoisomer thereof, or a solvate of the compound, the pharmaceutically acceptable salt or the stereoisomer according to any one of claims 1-9, or the antibody-drug conjugate, a pharmaceutically acceptable salt thereof, a stereoisomer thereof, or a solvate of the conjugate, the pharmaceutically acceptable salt or the stereoisomer according to claim 10 in preparation of a medicament, which is used for treatment of a disease associated with an abnormal cell activity, such as a cancer disease.

15. A pharmaceutical preparation, comprising the compound, a pharmaceutically acceptable salt thereof, a stereoisomer thereof, or a solvate of the compound, the pharmaceutically acceptable salt or the stereoisomer according to any one of claims 1-9, or the antibody-drug conjugate, a pharmaceutically acceptable salt thereof, a stereoisomer thereof, or a solvate of the conjugate, the pharmaceutically acceptable salt or the stereoisomer according to claim 10.

16. Use of the compound, a pharmaceutically acceptable salt thereof, a stereoisomer thereof, or a solvate of the compound, the pharmaceutically acceptable salt or the stereoisomer according to any one of claims 1-9, or the antibody-drug conjugate, a pharmaceutically acceptable salt thereof, a stereoisomer thereof, or a solvate of the conjugate, the pharmaceutically acceptable salt or the stereoisomer according to claim 10, or the pharmaceutical composition according to claim 13 in preparation of a pharmaceutical preparation.

17. A kit, comprising the compound, a pharmaceutically acceptable salt thereof, a stereoisomer thereof, or a solvate of the compound, the pharmaceutically acceptable salt or the stereoisomer according to any one of claims 1-9, or the antibody-drug conjugate, a pharmaceutically acceptable salt thereof, a stereoisomer thereof, or a solvate of the conjugate, the pharmaceutically acceptable salt or the stereoisomer according to claim 10, the pharmaceutical composition according to claim 13, or the pharmaceutical preparation according to claim 15.
